(19) Europäisches Patentamt
European Patent Office
Office européen des brevets

(11) Veröffentlichungsnummer: **0 203 488**
**A2**

(12) **EUROPÄISCHE PATENTANMELDUNG**

(21) Anmeldenummer: **86106777.5**

(22) Anmeldetag: **17.05.86**

(51) Int. Cl.⁴: **C 07 D 401/04**, C 07 D 471/04, C 07 D 215/56, A 61 K 31/47, A 61 K 31/435 // (C07D471/04, 221:00, 221:00)

(30) Priorität: **30.05.85 DE 3519286**

(43) Veröffentlichungstag der Anmeldung: **03.12.86 Patentblatt 86/49**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Schriewer, Michael, Dr., Heymannstrasse 36, D-5090 Leverkusen 1 (DE)**
Erfinder: **Grohe, Klaus, Dr., Am Wasserturm 10, D-5068 Odenthal (DE)**
Erfinder: **Zeiler, Hans-Joachim, Dr., Elsbeeker Strasse 46, D-5620 Velbert 15 (DE)**
Erfinder: **Metzger, Karl-Georg, Dr., Pahlkestrasse 3, D-5600 Wuppertal (DE)**

(54) **7-Azolyl-1-cyclopropyl-1,4-dihydro-4-oxo-3-chinolin-carbonsäuren und -1,8-napthyridin-3-carbonsäuren, Verfahren zu ihrer Herstellung sowie diese enthaltende antibakterielle Mittel.**

(57) Die vorliegende Erfindung betrifft neue 7-Azolyl-1-cyclopropyl-1,4-dihydro-4-oxo-3-chinolincarbonsäuren und -1,8-naphthyridin-3-carbonsäuren und ihre pharmazeutisch verwendbaren Hydrate, Salze und Ester, Verfahren zu ihrer Herstellung sowie diese enthaltende antibakterielle Mittel.

EP 0 203 488 A2

BAYER AKTIENGESELLSCHAFT 5090 Leverkusen, Bayerwerk
Konzernverwaltung RP
Patentabteilung Ad/by-c

# 7-Azolyl-1-cyclopropyl-1,4-dihydro-4-oxo-3-chinolincarbonsäuren und -1,8-naphthyridin-3-carbonsäuren, Verfahren zu ihrer Herstellung sowie diese enthaltende antibakterielle Mittel

Die vorliegende Erfindung betrifft neue 7-Azolyl-1-cyclopropyl-1,4-dihydro-4-oxo-3-chinolincarbonsäuren und -1,8-naphthyridin-3-carbonsäuren und ihre pharmazeutisch verwendbaren Hydrate, Salze und Ester, Verfahren zu ihrer Herstellung sowie diese enthaltende antibakterielle Mittel.

Aus der europäischen Patentanmeldung 115 049 ist bereits bekannt geworden, daß 1-Ethyl-6,8-difluor-1,4-dihydro-7-(1-imidazolyl)-4-oxo-3-chinolincarbonsäure antibakteriell wirksam ist.

Es wurde nun gefunden, daß die neuen Carbonsäuren der Formel (I),

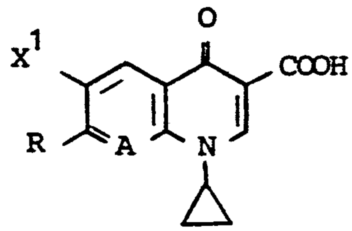

(I)

in welcher

$X^1$ für Halogen, wie Fluor, Chlor oder Brom, oder Nitro steht,

A für Stickstoff oder C-$X^2$ steht, worin

$X^2$ Wasserstoff oder Halogen, wie Fluor, Chlor oder Brom bedeutet, und

R für einen über Stickstoff gebundenen, gegebenenfalls durch Alkyl mit 1 bis 4 Kohlenstoffatomen, Alkoxy mit 1 bis 4 Kohlenstoffatomen, Phenyl, Halogen, wie Fluor, Chlor oder Brom, Nitro, Amino, Hydroxy, Cyano, Ethoxycarbonyl oder Carboxy ein- bis dreifach substituierten Azolyl-Rest, wie für einen Pyrrolyl-, Pyrazolyl-, Imidazolyl-, 1,2,3-Triazolyl- oder 1,2,4-Triazolyl-Rest steht,

und deren pharmazeutisch verwendbaren Hydrate, Säureadditionssalze, Alkali- und Erdalkalisalze, sowie deren Prodrug-Formen, wie Methyl-, Ethyl- oder (2-Methoxyethyl)-ester, eine hohe antibakterielle Wirkung aufweisen.

Bevorzugt sind diejenigen Verbindungen der Formel (I), in denen

$X^1$ für Fluor, Chlor oder Nitro steht,

A für Stickstoff oder $C-X^2$ steht, worin

$X^2$ Wasserstoff, Fluor oder Chlor bedeutet, und

R für einen über Stickstoff gebundenen, gegebenenfalls durch Methyl, Ethyl, Alkoxy mit 1 bis 3 Kohlenstoffatomen, Phenyl, Chlor, Nitro, Amino, Hydroxy oder Ethoxycarbonyl ein- oder zweifach substituierten Azolyl-Rest, wie für einen Pyrrolyl-, Pyrazolyl-, Imidazolyl-, 1,2,3-Triazolyl- oder 1,2,4-Triazolyl-Rest, steht,

und deren pharmazeutisch verwendbaren Hydrate, Säureadditionssalze, Alkali- und Erdalkalisalze, sowie deren Prodrug-Formen, wie Methyl-, Ethyl- oder (2-Methoxyethyl)-ester.

Besonders bevorzugt sind diejenigen Verbindungen der Formel (I), in denen

$X^1$ für Fluor, Chlor oder Nitro steht,

A für $C-X^2$ steht, worin

$X^2$ Wasserstoff, Fluor oder Chlor bedeutet, und

R für einen über Stickstoff gebundenen, gegebenenfalls durch Methyl, Alkoxy mit 1 bis 3 Kohlenstoffatomen,

Phenyl, Chlor, Nitro, Amino, Hydroxy oder Ethoxycarbonyl ein- oder zweifach substituierten Azolyl-Rest, wie für einen Pyrrolyl-, Pyrazolyl-, Imidazolyl-, 1,2,3-Triazolyl- oder 1,2,4-Triazolyl-Rest, steht,

und deren pharmazeutisch verwendbaren Hydrate, Säureadditionssalze, Alkali- und Erdalkalisalze, sowie deren Prodrug-Formen, wie Methyl-, Ethyl- oder (2-Methoxyethyl)-ester.

Weiterhin wurde gefunden, daß man die Verbindungen der Formel (I) erhält, wenn man eine Verbindung der Formel (II),

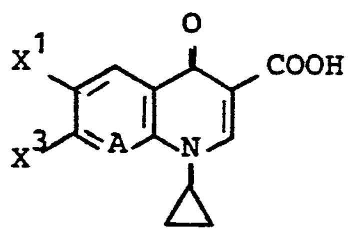

(II)

in welcher

A und $X^1$ die oben angegebene Bedeutung haben und

$X^3$ für Fluor oder Chlor steht,

mit Azolen der Formel (III),

R - H (III)

in welcher

R die oben angegebene Bedeutung hat,

gegebenenfalls in Gegenwart von Säurebindern umsetzt (Methode A).

Erfindungsgemäße Verbindungen der Formel (I), in welcher R für einen gegebenenfalls substituierten über Stickstoff verknüpften Pyrrol-Rest steht, können auch erhalten werden, wenn man eine Verbindung der Formel (IV)

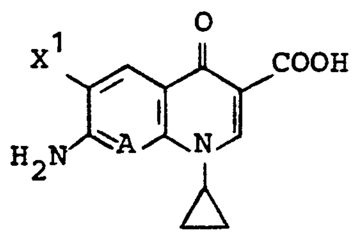

(IV)

in welcher

A und $X^1$ die oben angegebene Bedeutung haben,

mit 1,4-Dioxoverbindungen der Formel (V),

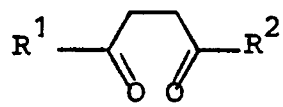

(V)

in welcher

$R^1$ und $R^2$ gleich oder verschieden sind und für Wasserstoff, Methyl oder Ethyl stehen,

bzw. mit verkappten 1,4-Dioxoverbindungen der Formel (VI)

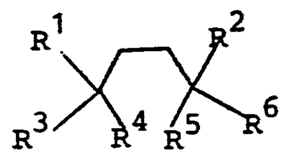

(VI)

in welcher

$R^1$ und $R^2$ die oben angegebene Bedeutung haben,

$R^3$,$R^4$,$R^5$ und $R^6$ gleich oder verschieden sind und für Methoxy, Ethoxy oder Chlor stehen oder $R^4$ und $R^5$ gemeinsam eine Sauerstoffbrücke bilden können,

umsetzt (Methode B).

In diese Reaktion können auch Methyl-, Ethyl- oder (2-Methoxyethyl)-ester der Verbindungen (IV) eingesetzt werden und die Umsetzungsprodukte gegebenenfalls unter sauren Bedingungen zu den erfindungsgemäßen Verbindungen der Formel (I), in welcher R für einen gegebenenfalls substituierten über Stickstoff verknüpften Pyrrol-Ring steht, verseift werden.

Erfindungsgemäße Verbindungen der Formel (I), in welcher R für einen über Stickstoff verknüpften Pyrazolrest steht, können auch erhalten werden, wenn man eine Hydrazinocarbonsäure der Formel (VII),

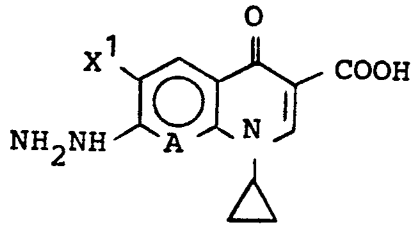

(VII)

in welcher

A und $X^1$ die oben angegebene Bedeutung haben,

mit 1,3-Dicarbonylverbindungen der Formel (VIII),

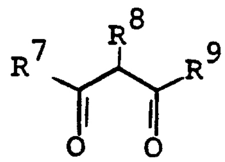

(VIII)

in welcher

$R^7$,$R^8$ und $R^9$ gleich oder verschieden sind und für Wasserstoff, Methyl, Ethyl, Methoxy oder Ethoxy stehen,

bzw. mit verkappten 1,3-Dicarbonylverbindungen der Formel (IX),

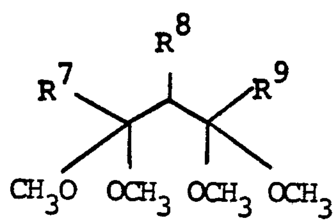

(IX)

in welcher

$R^7$,$R^8$ und $R^9$ die oben angegebene Bedeutung haben,

0203488

oder mit verkappten 1,3-Dicarbonylverbindungen der Formel (X),

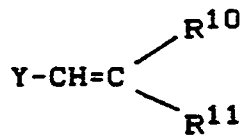

(X)

in welcher

Y für Dimethylamino, Methoxy oder Ethoxy,

$R^{10}$ für Formyl, Methoxycarbonyl, Ethoxycarbonyl oder Cyano und

$R^{11}$ für Wasserstoff, Alkyl mit 1 bis 4 Kohlenstoffatomen, Alkoxy mit 1 bis 4 Kohlenstoffatomen, Methoxycarbonyl, Ethoxycarbonyl oder Cyano steht, umsetzt (Methode C).

Verwendet man bei der Umsetzung nach Methode A 1-Cyclopropyl-6,7-difluor-1,4-dihydro-4-oxo-3-chinolincarbonsäure und Pyrazol als Ausgangsstoffe, so kann der Reaktionsablauf durch das folgende Formelschema wiedergegeben werden:

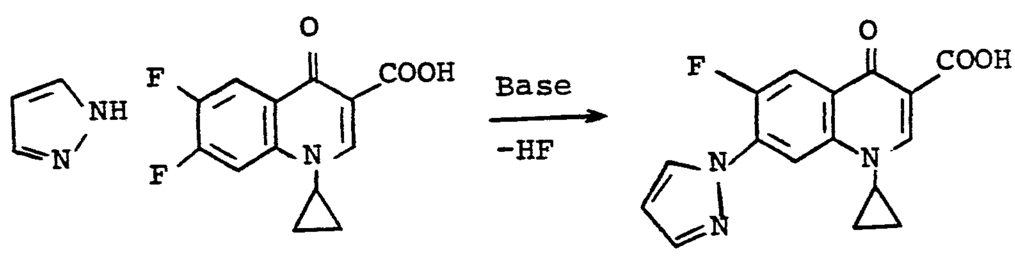

Verwendet man beispielsweise bei der Umsetzung nach Methode B 2,5-Dimethoxy-tetrahydrofuran und 7-Amino-1-cyclopropyl-6,8-difluor-1,4-dihydro-4-oxo-3-chinolincarbonsäure als Ausgangsstoffe, so kann der Reaktionsablauf durch das folgende Formelschema wiedergegeben werden:

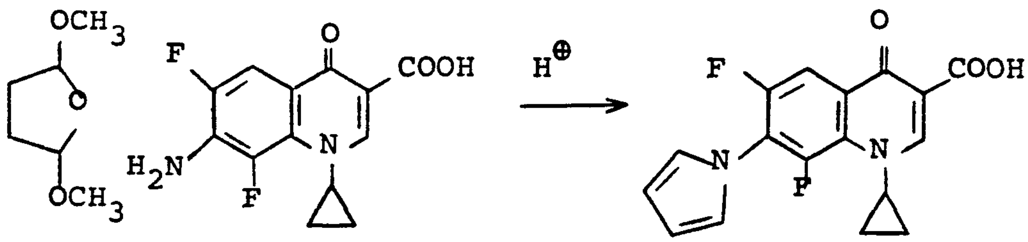


Verwendet man beispielsweise bei der Umsetzung nach Methode C 1,1,3,3-Tetramethoxypropan und 1-Cyclopropyl-6-fluor-7-hydrazino-1,4-dihydro-4-oxo-3-chinolincarbonsäure als Ausgangsstoffe, so kann der Reaktionsablauf durch das folgende Formelschema wiedergegeben werden:

$(CH_3O)_2CH-CH_2-CH(OCH_3)_2$ +

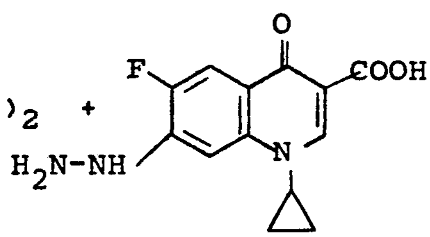

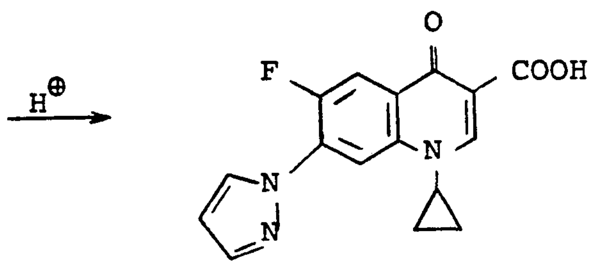

Obwohl die erfindungsgemäßen Verbindungen mit einem Hydroxy- oder Aminoazolyl-Rest als Substituenten im tautomeren Gleichgewicht überwiegend in der Oxo- bzw. Imino-Form vorliegen können, werden die Strukturen auch in diesen Fällen als Hydroxy- bzw. Amino-Form formuliert; z.B.

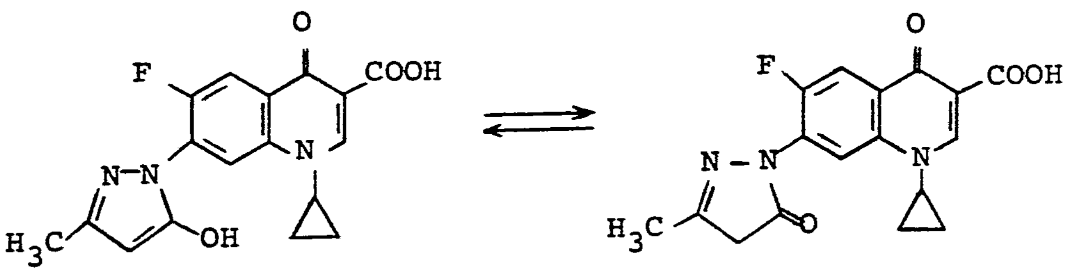

"Hydroxy-Form" "Oxo-Form"

Die als Ausgangsstoffe nach Methode A verwendeten 1-Cyclopropyl-1,4-dihydro-4-oxo-3-chinolincarbonsäuren [(II), A = C-$X^2$] (DE-OS 31 42 854, DE-OS 33 18 145) und 1-Cyclopropyl-1,4-dihydro-4-oxo-1,8-naphthyridin-3-carbonsäuren [(II), A = N] (EP 132 845) sind aus der Patentliteratur bekannt oder können gemäß folgendem Formelschema hergestellt werden:

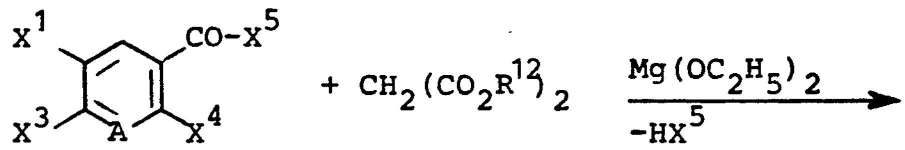


(1) (2)

$X^3$, $X^4$ = Cl, F, $NO_2$ $R^{12}$ = $CH_3$, $C_2H_5$

$X^5$ = Cl, F

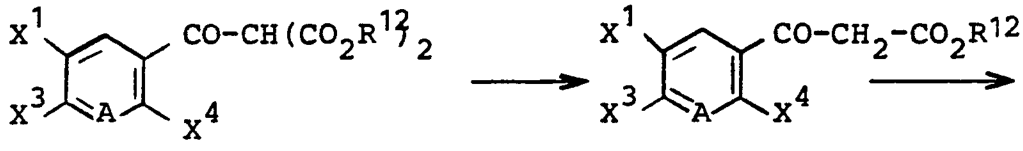

(3) (4)

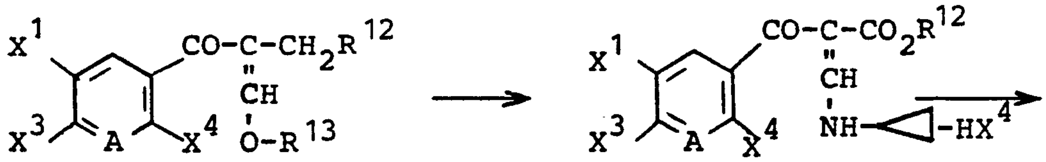


(5) (6)

$R^{13}$ = $CH_3$, $C_2H_5$

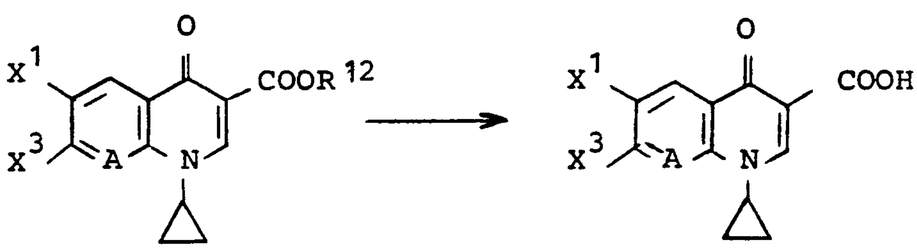

(7) (II)

Danach wird Malonsäuredialkylester (2) in Gegenwart von Magnesiumethylat mit dem entsprechenden Carbonsäurefluorid bzw. -chlorid (1) zum Acylmalonester (3) acyliert (Organicum, 3. Auflage, 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Acylessigsäurealkylester (4), der mit Orthoameisensäuretrialkylester/Acetanhydrid in den entsprechenden 2-Acyl-3-alkoxy-acrylsäure-alkylester (5) übergeht. Die Umsetzung von (5) mit Cyclopropylamin in einem Lösungsmittel, wie z.B. Methylenchlorid, Chloroform, Methanol, Ethanol, Cyclohexan oder Toluol führt in leicht exothermer Reaktion zum gewünschten Zwischenprodukt (6).

Die Cyclisierungsreaktion (6) ⟶ (7) wird in einem Temperaturbereich von etwa 60 bis 300°C, bevorzugt 80 bis 180°C durchgeführt.

Als Verdünnungsmittel können Dioxan, Dimethylsulfoxid, N-Methylpyrrolidon, Sulfolan, Hexamethylphosphorsäuretrisamid und bevorzugt N,N-Dimethylformamid verwendet werden.

Als Säurebinder kommen für diese Reaktionsstufe Kalium-tert.-butanolat, Butyl-lithium, Lithium-phenyl, Phenylmagnesiumbromid, Natriummethylat, Natriumhydrid, Natrium- oder Kaliumcarbonat in Betracht. Wenn bei dieser Cyclisierungsreaktion Fluorwasserstoff abgespalten werden soll, verwendet man besonders bevorzugt Kalium- oder Natriumfluorid. Es kann von Nutzen sein, einen Überschuß von 10 Mol-% an Base einzusetzen.

Die anschließende Esterhydrolyse von (7) unter basischen oder sauren Bedingungen führt zu den Carbonsäuren (II).

Einige der als Ausgangsstoffe für diesen Syntheseweg verwendeten Benzoylhalogenide (1) sind neu und werden wie folgt hergestellt:

3,5-Dichlor-2,4-difluor-benzoylfluorid (Siedepunkt 97°C/20 mbar; $n_D^{20}$ = 1,5148) und 5-Chlor-2,3,4-trifluorbenzoylfluorid (Siedepunkt 68 - 70°/20 mbar; $n_D^{20}$ = 1,4764) erhält man nebeneinander beim Erhitzen von Tetrachlorbenzoylchlorid mit Kaliumfluorid in Sulfolan auf erhöhte Temperaturen:

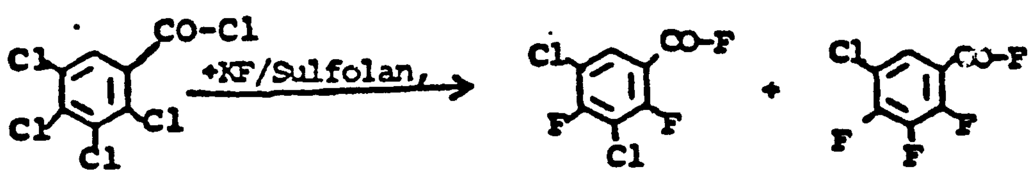


Die Chlorierung von 2,4,5-Trifluorbenzoesäure in Chlorsulfonsäure führt zur 3-Chlor-2,4,5-trifluorbenzoesäure, die als Rohprodukt mit Thionylchlorid zum 3-Chlor-2,4,5-trifluorbenzoylchlorid (Siedepunkt 94°C/18 mbar; $n_D^{20}$ = 1,5164) umgesetzt wird.

0203488

Die als Ausgangsverbindungen verwendeten Azole der Formel (III) sind bekannt. Als Beispiele seien genannt: Pyrrol, 2-Methylpyrrol, 3-Methylpyrrol, 2,5-Dimethylpyrrol, Pyrazol, 3-Methylpyrazol, 3,5-Dimethylpyrazol, 4-Chlorpyrazol, 4-Nitropyrazol, 2-Methylimidazol, 2-Phenylimidazol, 4-Nitroimidazol, 4-Imidazolcarbonsäureethylester, 2,4-Dimethylimidazol, 4,5-Dimethylimidazol, 1,2,3-Triazol, 1,2,4-Triazol.

Die als Ausgangsverbindungen nach Methode B verwendeten Aminocarbonsäuren der Formel (IV) sind neu und können durch Umsetzung der entsprechenden Halogencarbonsäuren der Formel (II),

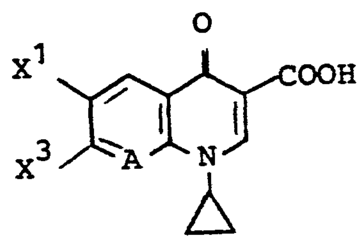

(II)

in welcher

A, $X^1$ und $X^3$ die oben angegebene Bedeutung haben,

mit Ammoniak in einem Druckgefäß erhalten werden. Man verwendet hierfür Lösungsmittel wie Dimethylformamid, Dimethylsulfoxid, Pyridin oder einen Alkohol wie Methanol, Ethanol, n-Propanol, Isopropanol oder Glykolmonomethylether oder auch Gemische dieser Lösungsmittel. Die Reaktionstemperaturen liegen im allgemeinen zwischen etwa 80° und 200°, vorzugsweise zwischen 140° und 170°. Als Beispiele für Verbindungen der Formel (IV) seien genannt:

7-Amino-1-cyclopropyl-6-fluor-1,4-dihydro-4-oxo-3-chinolincarbonsäure,
7-Amino-6-chlor-1-cyclopropyl-1,4-dihydro-4-oxo-3-chinolincarbonsäure,
7-Amino-1-cyclopropyl-1,4-dihydro-6-nitro-4-oxo-3-chinolincarbonsäure,
7-Amino-6-chlor-1-cyclopropyl-8-fluor-1,4-dihydro-4-oxo-3-chinolincarbonsäure,
7-Amino-8-chlor-1-cyclopropyl-6-fluor-1,4-dihydro-4-oxo-3-chinolincarbonsäure,
7-Amino-6,8-dichlor-1-cyclopropyl-1,4-dihydro-4-oxo-3-chinolincarbonsäure,
7-Amino-1-cyclopropyl-6,8-difluor-1,4-dihydro-4-oxo-3-chinolincarbonsäure,
7-Amino-1-cyclopropyl-6-fluor-1,4-dihydro-4-oxo-1,8-naphthyridin-3-carbonsäure.

Die als Ausgangsverbindungen verwendeten 1,4-Dioxoverbindungen der Formel (V) sind bekannt. Als Beispiele seien genannt: Butandial, 2,5-Hexandion, 4-Hexanon-1-al, 2,5-Heptandion, 3-Methyl-2,5-hexandion.

Die als Ausgangsverbindungen verwendeten Verbindungen der Formel (VI) sind bekannt. Als Beispiele seien genannt: 2,5-Dimethoxytetrahydrofuran, 1,1,4,4-Tetramethoxybutan, 1,4-Dichlor-1,4-dimethoxy-butan.

Die als Ausgangsstoffe nach Methode C verwendeten Hydrazinocarbonsäuren (VII) sind neu und können durch Umsetzung der entsprechenden Halogencarbonsäuren der Formel (II),

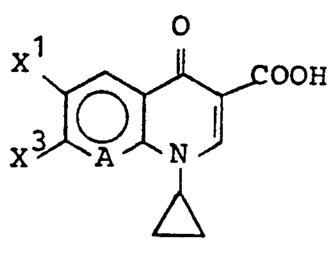

(II)

in welcher

A, $X^1$ und $X^3$ die oben angegebene Bedeutung haben,

mit Hydrazin oder Hydrazinhydrat erhalten werden. Man verwendet hierfür Lösungsmittel wie Dimethylformamid, Dimethylsulfoxid, Pyridin oder einen Alkohol wie Methanol, Ethanol, n-Propanol, Isopropanol oder Glykolmonomethylether oder Gemische dieser Lösungsmittel. Die Reaktionstemperaturen liegen im allgemeinen zwischen etwa 50° und 200°, vorzugsweise zwischen 80° und 150°.

Als Beispiele für Verbindungen der Formel (VII) seien genannt:

1-Cyclopropyl-6-fluor-7-hydrazino-1,4-dihydro-4-oxo-3-chinolincarbonsäure,
6-Chlor-1-cyclopropyl-7-hydrazino-1,4-dihydro-4-oxo-3-chinolincarbonsäure,
1-Cyclopropyl-7-hydrazino-1,4-dihydro-6-nitro-4-oxo-3-chinolincarbonsäure,
6-Chlor-1-cyclopropyl-8-fluor-7-hydrazino-1,4-dihydro-4-oxo-3-chinolincarbonsäure,
8-Chlor-1-cyclopropyl-6-fluor-7-hydrazino-1,4-dihydro-4-oxo-3-chinolincarbonsäure,
6,8-Dichlor-1-cyclopropyl-7-hydrazino-1,4-dihydro-4-oxo-3-chinolincarbonsäure,
1-Cyclopropyl-6,8-difluor-7-hydrazino-1,4-dihydro-4-oxo-3-chinolincarbonsäure,
1-Cyclopropyl-6-fluor-7-hydrazino-1,4-dihydro-4-oxo-1,8-naphthyridin-3-carbonsäure.

Die als Ausgangsverbindungen verwendeten 1,3-Dicarbonylverbindungen der Formel (VIII) sind bekannt. Als Beispiele seien genannt: Acetessigsäuremethylester, Acetessigsäureethylester, Acetylaceton, Malondialdehyd.

Die als Ausgangsverbindungen verwendeten verkappten 1,3-Dicarbonylverbindungen der Formel (IX) sind bekannt. Als Beispiele seien genannt: 1,1,3,3-Tetramethoxypropan, 1,1,3,3-Tetramethoxy-2-methyl-propan.

Die als Ausgangsstoffe verwendeten verkappten 1,3-Dicarbonylverbindungen der Formel (X) sind ebenfalls bekannt. Als Beispiele seien genannt: 2-Dimethylamino-1-methylacrolein, Ethoxymethylenmalonsäurediethylester, Ethoxymethylenmalonsäuredinitril.

Die Umsetzung von (II) mit (III) gemäß Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet oder auch in einer Schmelze aus überschüssigem Azol gearbeitet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo[2,2,2]-octan (DABCO), 1,8-Diazabicyclo[5,4,0]undec-7-en (DBU) oder überschüssiges Azol (III).

Die Reaktionstemperaturen können in einem größeren Bereich variiert werden. Im allgemeinen arbeitet man zwischen etwa 70 und 200°C, vorzugsweise zwischen 100 und 180°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II) 1 bis 50 Mol, vorzugsweise 1 bis 30 Mol des Azols (III) ein.

Die Umsetzung von (IV) mit (V) bzw. (VI) wird vorzugsweise unter sauren Bedingungen in einem Verdünnungsmittel wie Essigsäure, Methylenchlorid, Chloroform oder Acetonitril durchgeführt. Als Katalysator kann beispielsweise ein saurer Ionenaustauscher, Schwefelsäure, Chlorwasserstoffsäure, Phosphorsäure oder p-Toluolsulfonsäure zugesetzt werden.

Die Reaktionstemperaturen können in einem größeren Bereich variiert werden. Im allgemeinen arbeitet man zwischen etwa 0° und 180°C, vorzugsweise zwischen 70° und 150°C.

Die Umsetzung kann bei Normaldruck, aber auch bei erhöhtem Druck durchgeführt werden. Im allgemeinen arbeitet man bei Normaldruck.

Bei der Durchführung des erfindungsgemäßen Verfahrens setzt man auf 1 Mol der Verbindung (IV) 1 bis 5 Mol, vorzugsweise 1 bis 2,5 Mol der Verbindung (V) bzw. (VI) ein.

Die Umsetzung von (VII) mit (VIII), (IX) oder (X) gemäß Methode C wird vorzugsweise in einem Verdünnungsmittel wie Dimethylsulfoxid, N,N-Dimethylformamid, Essigsäure, Wasser, einem Alkohol wie Methanol, Ethanol, n-Propanol, Isopropanol oder Glykolmonomethylether vorgenommen. Ebenso können Gemische dieser Lösungsmittel verwendet werden.

Als Katalysatoren können alle üblichen Protonensäuren verwendet werden. Als besonders geeignet seien genannt: Chlorwasserstoffsäure, Schwefelsäure, Phosphorsäure und p-Toluolsulfonsäure.

Die Reaktionstemperaturen können im weiten Bereich variiert werden. Im allgemeinen arbeitet man zwischen etwa 20° und 150°, vorzugsweise zwischen 50° und 120°.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol Carbonsäure (VII) 1 bis 6 Mol, vorzugsweise 1 bis 3 Mol einer Verbindung (VIII), (IX) oder (X) ein.

Die Herstellung der Säureadditionssalze der erfindungsgemäßen Verbindungen mit einem ausreichend basischen Azolyl-Rest erfolgt in üblicher Weise z.B. durch Lösen des Betains in überschüssiger wäßriger Säure und Ausfällen des Salzes mit einem mit Wasser mischbaren organischen Lösungsmittel (Methanol, Ethanol, Aceton, Acetonitril).

Man kann auch äquivalente Mengen Betain und Säure in Wasser bis zur Lösung erhitzen und anschließend bis zur Trockne eindampfen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, Galacturonsäure, Gluconsäure, Glutaminsäure, Embonsäure oder Asparaginsäure zu verstehen.

Die Alkali- oder Erdalkalisalze werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze.

Außer den in den Beispielen aufgeführten Verbindungen seien als neue Wirkstoffe im einzelnen genannt:
6-Chlor-1-cyclopropyl-8-fluor-1,4-dihydro-4-oxo-7-(1-pyrrolyl)-3-chinolincarbonsäure,
8-Chlor-1-cyclopropyl-6-fluor-1,4-dihydro-4-oxo-7-(1-pyrrolyl)-3-chinolincarbonsäure,
6,8-Dichlor-1-cyclopropyl-1,4-dihydro-4-oxo-7-(1-pyrrolyl)-3-chinolincarbonsäure,
1-Cyclopropyl-6-fluor-1,4-dihydro-4-oxo-7-(1-pyrrolyl)-1,8-naphthyridin-3-carbonsäure,
6-Chlor-1-cyclopropyl-1,4-dihydro-4-oxo-7-(1-pyrrolyl)-1,8-naphthyridin-3-carbonsäure,
6-Chlor-1-cyclopropyl-1,4-dihydro-7-(1-imidazolyl)-4-oxo-1,8-naphthyridincarbonsäure.

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die

folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephri-

tis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Metritis-Mastitis-Agalaktiae-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder ge-

0203488

heilt werden und dadurch eine Förderung des Wachstums und eine Verbesserung der Verwertung des Futters erreicht werden.

In der nachstehenden Tabelle sind die MHK-Werte einiger der erfindungsgemäßen Verbindungen im Vergleich zu Ciprofloxacin angegeben.

MHK-Werte (mcg/ml)

| Stamm | EP 115 049*) | Verbindung aus Beispiel 21 | Beispiel 20 | Beispiel 2 | Beispiel 1 |
|---|---|---|---|---|---|
| E.coli Neum. | 0.125 | 0.03 | 0.03 | 0.25 | 0.06 |
| E.coli T 7 | 0.125 | 0.03 | 0.03 | 0.125 | 0.06 |
| Klebsiella pneum. 63 | 0.5 | 0.06 | 0.25 | 0.5 | 0.125 |
| Klebsiella pneum. 8085 | 0.25 | 0.03 | 0.03 | 0.125 | 0.06 |
| Proteus vulg. 1017 | 0.5 | 0.125 | 0.25 | 0.5 | 0.25 |
| Staph. aur. FK 422 | 0.5 | 0.125 | 0.03 | 0.06 | 0.03 |
| Staph. aur. 1756 | 1 | 0.125 | 0.03 | 0.06 | 0.03 |
| Staph. aur. 133 | 1 | 0.125 | 0.03 | 0.06 | 0.03 |
| Streptococcus faec.27101 | 2 | 0.5 | 0.125 | 0.25 | 0.25 |
| Pseudom.aerug.Ellsw. | 1 | 0.25 | 0.5 | 1.0 | 0.5 |

Agardilutionstest / Isosensitestmedium

*) 1-Ethyl-6,8-difluor-1,4-dihydro-7-(1-imidazolyl)-4-oxo-3-chinolincarbonsäure

Beispiel für eine erfindungsgemäße Tablette

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN Polyethylenglykole (DAB) | 2,0 mg |
| Titan-(IV)-oxid | 2,0 mg |
| | 10,0 mg |

0203488

## Beispiel A

6-Chlor-1-cyclopropyl-7,8-difluor-1,4-dihydro-4-oxo-3-chinolincarbonsäure

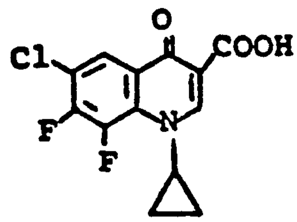

15,7 g (0,65 mol) Magnesiumspäne werden in 40 ml Ethanol und 2 ml Tetrachlormethan verrührt und nach begonnener Reaktion bei 50 - 60°C tropfenweise mit 103 g (0,64 mol) Malonsäurediethylester in 80 ml Ethanol und 250 ml Toluol versetzt. Man rührt 1 Stunde bei dieser Temperatur nach, kühlt auf -5 bis -10°C, tropft eine Lösung von 138 g (0,65 mol) 5-Chlor-2,3,4-trifluorbenzoylfluorid in 63 ml Toluol zu, rührt noch 1 Stunde bei 0° und läßt über Nacht bei Raumtemperatur stehen. Danach wird noch 2 Stunden auf 40-50°C erwärmt, abgekühlt und mit 250 ml Eiswasser und 38,5 ml konzentrierter Schwefelsäure versetzt. Die organische Phase wird abgetrennt, die wäßrige Phase mit 2 mal 150 ml Toluol extrahiert, die vereinigten organischen Phasen mit gesättigter Natriumchlorid-Lösung gewaschen, mit Natriumsulfat getrocknet und eingeengt.

Der Rückstand wird mit 200 ml Wasser versetzt (vorteilhaft ist hier die Zugabe von 0,4 g 4-Toluolsulfonsäure) und zur Desethoxycarbonylierung 5 Stunden unter Rückfluß erhitzt. Man extrahiert mit 3 mal 200 ml Dichlormethan,

wäscht mit gesättigter Natriumchlorid-Lösung, trocknet mit Natriumsulfat, engt ein und destilliert im Hochvakuum. Man erhält 103 g (56,5 %) (5-Chlor-2,3,4-trifluor-benzoyl)-essigsäure-ethylester mit einem Siedepunkt von 110°C/0,9 Torr.

103 g (0,37 mol) des erhaltenen Esters und 83 g (0,56 mol) Orthoameisensäuretriethylester werden mit 95 g Essigsäureanhydrid 2 Stunden auf 150-160°C erhitzt und anschließend bei 120-130°C unter Normaldruck, danach im Hochvakuum eingeengt. Man erhält 115 g (92 % der Theorie) 2-(5-Chlor-2,3,4-trifluor-benzoyl)-3-ethoxyacrylsäure-ethylester als Öl.

84,1 g (0,25 mol) dieser Verbindung werden in 170 ml Ethanol unter Eiskühlung tropfenweise mit 14,8 g (0,26 mol) Cyclopropylamin versetzt und 2 Stunden bei Raumtemperatur gerührt. Danach wird mit 170 ml Wasser verrührt, in Eis gekühlt, der ausgefallene Niederschlag abgesaugt, mit Wasser und wenig Methanol gewaschen und getrocknet. Es werden 47 g (54 %) 2-(5-Chlor-2,3,4-trifluor-benzoyl)-3-cyclopropylamino-acrylsäureethylester vom Schmelzpunkt 71-73°C erhalten. Nach dem $^1$H-NMR-Spektrum liegt ein cis-trans-Gemisch vor.

47 g (0,14 mol) dieser Verbindung werden in 230 ml Dimethylformamid mit 9,7 g (0,23 mol) Natriumfluorid 2 Stunden auf 160-170°C erhitzt. Das Reaktionsgemisch wird in 400 ml Eiswasser eingegossen, der Niederschlag abgesaugt, mit Wasser gewaschen und getrocknet. Man isoliert 44 g (99 %) 6-Chlor-1-cyclopropyl-7,8-difluor-1,4-dihydro-4-oxo-3-chinolincarbonsäure-ethylester vom Schmelzpunkt 169-172°C.

44 g (0,13 mol) des Chinolincarbonsäureesters werden in 300 ml Eisessig und 179 ml Wasser mit 33 ml konzentrierter Schwefelsäure versetzt und 2 Stunden auf 150°C erhitzt. Das Reaktionsgemisch wird in 400 ml Eiswasser eingerührt, der Niederschlag abgesaugt, mit Wasser gewaschen und getrocknet. Es werden 37 g (95 % der Theorie) 6-Chlor-1-cyclopropyl-7,8-difluor-1,4-dihydro-4-oxo-3-chinolincarbonsäure mit einem Schmelzpunkt von 200-204°C isoliert.

Beispiel B

8-Chlor-1-cyclopropyl-6,7-difluor-1,4-dihydro-4-oxo-3-chinolincarbonsäure

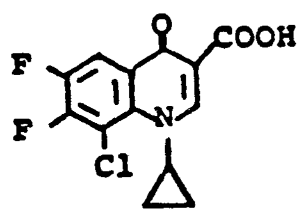

Man setzt 3-Chlor-2,4,5-trifluor-benzoylchlorid analog Beispiel A um, wobei folgende Stufen durchlaufen werden: (3-Chlor-2,4,5-trifluor-benzoyl)-essigsäure-ethylester als Enol (Ausbeute: 42 %, Schmelzpunkt 72 - 75°C), 2-(3-Chlor-2,4,5-trifluor-benzoyl)-3-ethoxy-acrylsäure-ethylester (Rohausbeute: 95 % Öl), 2-(3-Chlor-2,4,5-trifluor-benzoyl-3-cyclopropylamino-acrylsäureethylester (Ausbeute: 67 %, Schmelzpunkt 78-80°C), 8-Chlor-1-cyclopropyl-6,7-difluor-1,4-dihydro-4-oxo-3-chinolincarbonsäure-ethylester (Ausbeute: 85 %, Schmelzpunkt 154 - 157°C),

8-Chlor-1-cyclopropyl-6,7-difluor-1,4-dihydro-4-oxo-3-chinolincarbonsäure (Ausbeute: 97,6 %, Schmelzpunkt 189-192°C).

Beispiel C

6,8-Dichlor-1-cyclopropyl-7-fluor-1,4-dihydro-4-oxo-3-chinolincarbonsäure

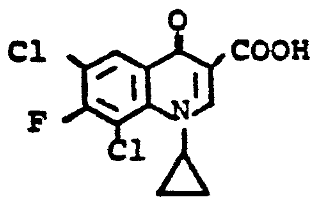

Man setzt 3,5-Dichlor-2,4-difluor-benzoylfluorid analog Beispiel A um, wobei folgende Stufen durchlaufen werden:

(3,5-Dichlor-2,4-difluor-benzoyl)-essigsäure-ethylester (Ausbeute: 43 %, Siedepunkt 133°C/2,5 Torr),
2-(3,5-Dichlor-2,4-difluor-benzoyl)-3-ethoxy-acrylsäure-ethylester (Rohausbeute: 91 % Öl),
2-(3,5-Dichlor-2,4-difluor-benzoyl)-3-cyclopropylamino-acrylsäureethylester (Ausbeute: 96 %, Schmelzpunkt 71-74°C),
6,8-Dichlor-1-cyclopropyl-7-fluor-1,4-dihydro-4-oxo-3-chinolincarbonsäure-ethylester (Ausbeute: 97 %, Schmelzpunkt 215-217°C unter Zersetzung),
6,8-Dichlor-1-cyclopropyl-7-fluor-1,4-dihydro-4-oxo-3-chinolincarbonsäure (Ausbeute: 93 %; Schmelzpunkt 204-206°C).

Beispiel D

1-Cyclopropyl-6,7-difluor-1,4-dihydro-4-oxo- 3-chinolin-carbonsäure

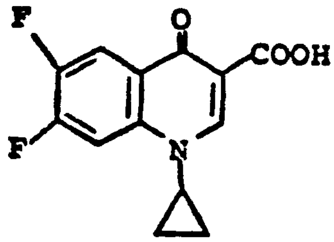

Ausgehend von 2,4,5-Trifluorbenzoylfluorid verfährt man analog Beispiel A, wobei folgende Stufen durchlaufen werden:

2,4,5-Trifluorbenzoyl-essigsäureethylester (Siedepunkt = 92-95°/0,5 mbar; Schmelzpunkt: 53-55°C), 2-(2,4,5-Trifluorbenzoyl)-3-cyclopropylaminoacrylsäureethylester (Öl), 1-Cyclopropyl-6,7-difluor-1,4-dihydro-4-oxo-3-chinolincarbonsäureethylester (Schmelzpunkt: 230-233°C), 1-Cyclopropyl-6,7-difluor-1,4-dihydro-4-oxo-3-chinolincarbonsäure (Schmelzpunkt: 302-303°C unter Zersetzung).

Beispiel E

6-Chlor-1-cyclopropyl-1,4-dihydro-7-nitro-4-oxo-3-chinolincarbonsäure

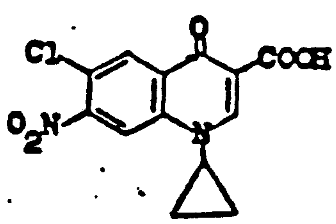

3,7 g Magnesiumspäne werden in 10 ml Ethanol und 1 ml Tetrachlormethan verrührt und nach begonnener Reaktion bei 50-60°C tropfenweise mit 24,6 g Malonsäurediethylester in 20 ml Ethanol und 80 ml Toluol versetzt. Man rührt 1 Stunde bei dieser Temperatur nach und tropft diese Lösung bei -5 bis -10°C zu einer Suspension von 40,8 g 5-Chlor-2,4-dinitrobenzoylchlorid in 50 ml Toluol. Man rührt noch 1 Stunde bei 0°C und läßt über Nacht bei Raumtemperatur stehen. Dann wird mit 80 ml Eiswasser und 10 ml konzentrierter Schwefelsäure versetzt. Die organische Phase wird abgetrennt, die wäßrige Phase mit 2 mal 50 ml Toluol extrahiert, die vereinigten organischen Phasen mit gesättigter Natriumchlorid-Lösung gewaschen, mit Natriumsulfat getrocknet und eingeengt. Der Rückstand (61,6 g) wird mit 100 ml Wasser versetzt (vorteilhaft ist hier die Zugabe von 0,3 g 4-Toluolsulfonsäure) und zur Hydrolyse und Decarboxylierung 3 Stunden unter Rückfluß erhitzt. Man saugt kalt ab und wäscht mit Petrolether nach. Es werden 47,5 g (5-Chlor-2,4-dinitro-benzoyl)-essigsäureethylester vom Schmelzpunkt 105-110°C erhalten.

47,5 g des erhaltenen Esters und 33,3 g Orthoameisensäure-triethylester werden mit 39 g Essigsäureanhydrid 3 Stunden auf 150-160°C erhitzt und anschließend bei 120-130°C unter Normaldruck, danach im Hochvakuum eingeengt. Man erhält 48,2 g 2-(5-Chlor-2,4-dinitro-benzoyl)-3-ethoxy-acrylsäure-ethylester als Öl.

48,2 g dieser Verbindung werden in 150 ml Ethanol unter Eiskühlung tropfenweise mit 7,4 g Cyclopropylamin versetzt und 1 Stunde bei Raumtemperatur gerührt. Danach wird mit

150 ml Wasser verrührt, mit Eis gekühlt, der ausgefallene Niederschlag abgesaugt, mit einem Ethanol-Wasser-Gemisch (1:1) gewaschen und getrocknet. Es werden 47,9 g 2-(5-Chlor-2,4-dinitro-benzoyl)-3-cyclopropylaminoacrylsäureethylester vom Schmelzpunkt 100-115°C erhalten. Nach der Umkristallisation aus Essigsäureethylester schmelzen die gelben Kristalle bei 130-132°C (31,4 g). Nach dem $^1$H-NMR-Spektrum liegt ein cis-trans-Gemisch vor.

3,8 g dieser Verbindung werden in 25 ml wasserfreiem Dioxan portionsweise mit 0,3 g 80 %igem Natriumhydrid versetzt und 2 Stunden unter Rückfluß zum Sieden erhitzt. Das Reaktionsgemisch wird vorsichtig auf Eis gegossen, der Niederschlag abgesaugt, mit Wasser gewaschen und getrocknet. Nach Umkristallisation aus Ethanol werden 2 g 6-Chlor-1-cyclopropyl-1,4-dihydro-7-nitro-4-oxo-3-chinolincarbonsäureethylester vom Schmelzpunkt 208-210°C erhalten.

1,7 g des Chinolincarbonsäureesters werden in 10 ml Eisessig und 8,5 ml Wasser mit 1,1 ml konzentrierter Schwefelsäure versetzt und 2 Stunden auf 150°C erhitzt. Das Reaktionsgemisch wird in 20 ml Eiswasser eingerührt, der Niederschlag abgesaugt, mit Wasser gewaschen und getrocknet. Es werden nach Umkristallisation aus Acetonitril 1,2 g 6-Chlor-1-cyclopropyl-1,4-dihydro-7-nitro-4-oxo-3-chinolincarbonsäure mit einem Schmelzpunkt von 248-250°C isoliert.

Beispiel F

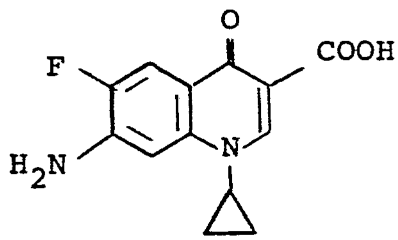

Eine Lösung von 15,9 g (0,06 mol) 1-Cyclopropyl-6,7-difluor-1,4-dihydro-4-oxo-3-chinolincarbonsäure in 180 ml Pyridin wird mit 100 ml einer gesättigten Lösung von Ammoniak in Ethanol versetzt. Man erhitzt diese Mischung während 15 Stunden auf 150°, engt im Vakuum ein, versetzt den Rückstand mit Wasser und säuert mit verdünnter Salzsäure (1:1) an. Der Niederschlag wird abgesaugt, mit Wasser gewaschen und bei 80°/12 mbar getrocknet. Man erhält 14,9 g (94,8 % der Theorie) 7-Amino-1-cyclopropyl-6-fluor-1,4-dihydro-4-oxo-3-chinolincarbonsäure vom Schmelzpunkt 288-290° (unter Zersetzung).

Beispiel G

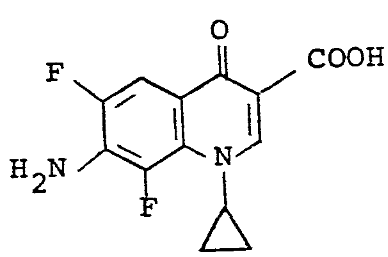

Man setzt 1-Cyclopropyl-6,7,8-trifluor-1,4-dihydro-4-oxo-3-chinolincarbonsäure analog Beispiel F mit ethanolischer Ammoniaklösung um und erhält 7-Amino-1-cyclopropyl-6,8-difluor-1,4-dihydro-4-oxo-3-chinolincarbonsäure vom Schmelzpunkt 314-317° (unter Zersetzung) (umkristallisiert aus Glykolmonomethylether).

Beispiel H

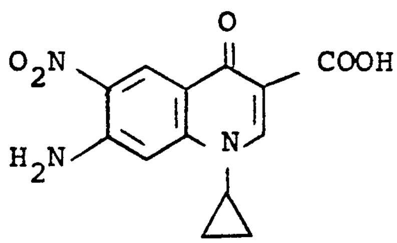

Eine Lösung von 9,6 g (0,03 mol) 7-Chlor-1-cyclopropyl-1,4-dihydro-6-nitro-4-oxo-3-chinolincarbonsäure in 100 ml Pyridin wird mit 30 ml einer gesättigten Lösung von Ammoniak in Ethanol versetzt und die Mischung 6 Stunden auf 130° erhitzt. Der Niederschlag wird nach dem Abkühlen abgesaugt, mit Wasser verrührt, mit verdünnter Salzsäure angesäuert, mit Wasser gewaschen und bei 100°/15 mbar getrocknet. Man erhält 4,1 g (47 % der Theorie) 7-Amino-1-cyclopropyl-1,4-dihydro-6-nitro-4-oxo-3-chinolincarbonsäure vom Schmelzpunkt 327-330° (unter Zersetzung).

Beispiel I

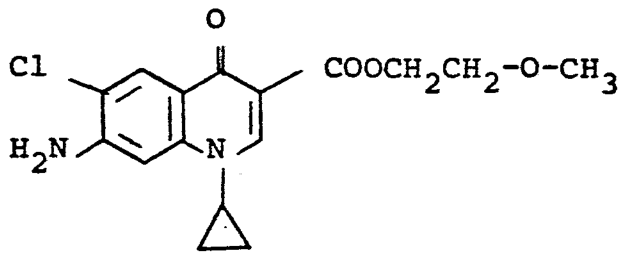

6,72 g (20 mmol) 6-Chlor-1-cyclopropyl-1,4-dihydro-7-nitro-4-oxo-3-chinolincarbonsäureethylester werden in 128 ml Eisessig mit 12,8 g Eisenfeilspäne 1 Stunde auf 80° erwärmt, heiß filtriert, mit heißem Eisessig gewaschen und das Filtrat bis auf etwa 50 ml eingeengt. Beim Abkühlen im Eisbad fällt ein Niederschlag aus, der nach 30 Minuten abgesaugt, mit Eiswasser gewaschen und bei 80° im Vakuum getrocknet wird. Man erhält 6 g (98 % der Theorie) 7-Amino-6-chlor-1-cyclopropyl-1,4-dihydro-4-oxo-3-chinolincarbonsäureethylester vom Schmelzpunkt 274-277°. Durch Umkristallisation dieser Verbindung aus Glykolmonomethylether/Ethanol (1:1) erhält man 4,5 g 7-Amino-6-chlor-1-cyclopropyl-1,4-dihydro-4-oxo-3-chinolincarbonsäure-(2-methoxyethyl)-ester vom Schmelzpunkt 253-254°.

Beispiel J

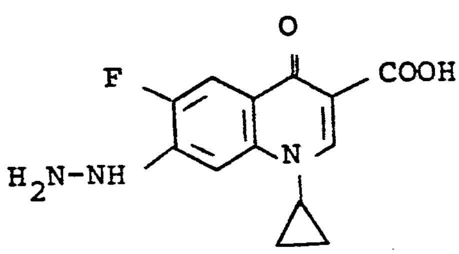

52,7 g (0,2 mol) 1-Cyclopropyl-6,7-difluor-1,4-dihydro-4-oxo-3-chinolincarbonsäure und 31,6 g 100 %iges Hydrazinhydrat werden in 350 ml Ethanol 8 Stunden gekocht. Nach Abkühlung versetzt man mit Wasser und saugt den Feststoff ab. Man erhält 52,5 g (95,7 % der Theorie) 1-Cyclopropyl-6-fluor-7-hydrazino-1,4-dihydro-4-oxo-3-chinolincarbonsäure vom Schmelzpunkt 287-289°.

Beispiel 1

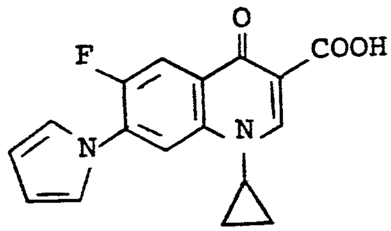

2,6 g (10 mmol) 7-Amino-1-cyclopropyl-6-fluor-1,4-dihydro-4-oxo-3-chinolincarbonsäure werden in 30 ml Essigsäure mit 3 g (22 mmol) 2,5-Dimethoxy-tetrahydrofuran versetzt und 1,5 Stunden unter Rückfluß erhitzt. Nach dem Abkühlen wird der Niederschlag abgesaugt, mit Methanol gewaschen und getrocknet. Man erhält 1,5 g (48 % der Theorie) 1-Cyclopropyl-6-fluor-1,4-dihydro-4-oxo-7-(1-pyrrolyl)-3-chinolincarbonsäure vom Schmelzpunkt 256-258°C.

Beispiel 2

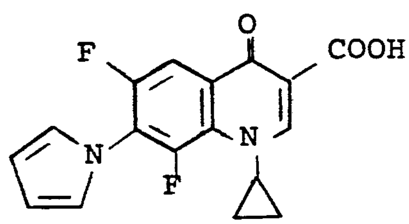

Man setzt 7-Amino-1-cyclopropyl-6,8-difluor-1,4-dihydro-4-oxo-3-chinolincarbonsäure analog Beispiel 1 mit 2,5-Dimethoxy-tetrahydrofuran um und erhält 1-Cyclopropyl-6,8-difluor-1,4-dihydro-4-oxo-7-(1-pyrrolyl)-3-chinolincarbonsäure vom Schmelzpunkt 262-272° (unter Zersetzung) (umkristallisiert aus Glykolmonomethylether).

Beispiel 3

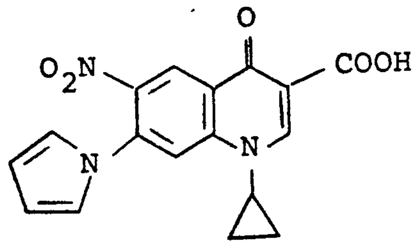

Man setzt 7-Amino-1-cyclopropyl-1,4-dihydro-6-nitro-4-oxo-3-chinolincarbonsäure analog Beispiel 1 mit 2,5-Dimethoxytetrahydrofuran während 5,5 Stunden bei 100° um und erhält so 1-Cyclopropyl-1,4-dihydro-6-nitro-4-oxo-7-(1-pyrrolyl)-3-chinolincarbonsäure vom Schmelzpunkt 242-245° (unter Zersetzung) (umkristallisiert aus Glykolmonomethylether).

Beispiel 4

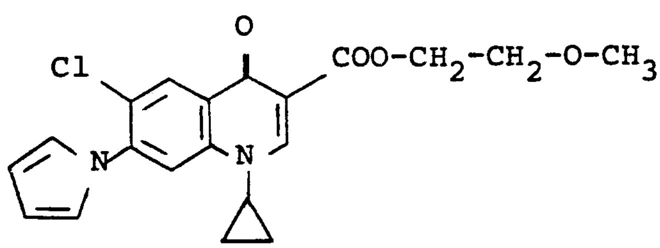

1,68 g (5,5 mmol)
7-Amino-1-cyclopropyl-6-chlor-1,4-dihydro-4-oxo-3-chinolincarbonsäure-(2-methoxyethyl)-ester werden in 15 ml

Essigsäure mit 1,5 g (11 mmol) 2,5-Dimethoxy-tetrahydrofuran 1,5 Stunden auf 100° erhitzt. Die erhaltene Suspension wird in 50 ml Wasser eingegossen, das Kristallisat abgesaugt, mit Wasser und Methanol gewaschen. Es werden 1,7 g (87 % der Theorie) 1-Cyclopropyl-6-chlor-1,4-dihydro-4-oxo-7-(1-pyrrolyl)-3-chinolincarbonsäure-(2-methoxyethyl)-ester vom Schmelzpunkt 202-206° erhalten.

Beispiel 5

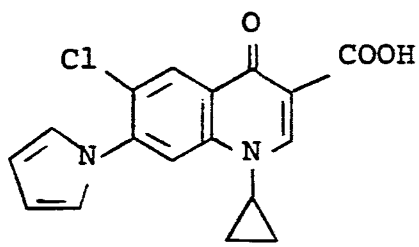

1,6 g (4,4 mmol) des Produktes aus Beispiel 4 werden in 10 ml Essigsäure mit 6 ml Wasser und 1 ml konzentrierter Schwefelsäure versetzt und 2 Stunden auf 150° erhitzt. Man gießt auf 50 ml Eiswasser aus, saugt den Niederschlag ab und wäscht mit Wasser und Methanol : 1,1 g vom Schmelzpunkt 182-185° (unter Zersetzung). Nach Umkristallisation aus Ethanol werden 0,8 g (55 % der Theorie) 6-Chlor-1-cyclopropyl-1,4-dihydro-4-oxo-7-(1-pyrrolyl)-3-chinolincarbonsäure vom Schmelzpunkt 202-206° (unter Zersetzung) erhalten.

Beispiel 6

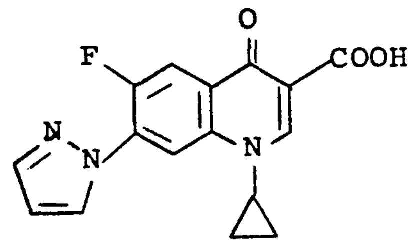

Methode A: 2,8 g (10,6 mmol) 1-Cyclopropyl-6,7-difluor-1,4-dihydro-4-oxo-3-chinolincarbonsäure werden in einer Schmelze von 30 g Pyrazol 4 Stunden auf 130° erhitzt, abgekühlt und mit 30 ml Wasser verrührt. Der ungelöste Rückstand wird abgesaugt, mit Wasser gewaschen und mit Methanol aufgekocht. Man isoliert 2,5 g (75,7 % der Theorie) 1-Cyclopropyl-6-fluor-1,4-dihydro-4-oxo-7-(1-pyrazolyl)-3-chinolincarbonsäure vom Schmelzpunkt 250-252° (unter Zersetzung).

Methode C: 1 g (3,6 mmol) Produkt aus Beispiel J, 0,73 g 1,1,3,3-Tetramethoxypropan und 1 ml verdünnter Salzsäure werden in 20 ml Ethanol eine Stunde gekocht. Nach Abkühlung auf Raumtemperatur wird der ausgefallene Feststoff abgesaugt. Ausbeute: 1,1 g (97,6 % der Theorie) 1-Cyclopropyl-6-fluor-1,4-dihydro-4-oxo-7-(1-pyrazolyl)-3-chinolincarbonsäure vom Schmelzpunkt 250-251°C (unter Zersetzung).

Beispiel 7

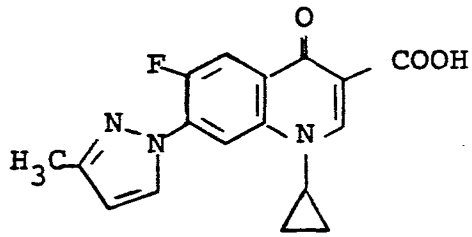

2,8 g (10,6 mmol) 1-Cyclopropyl-6,7-difluor-1,4-dihydro-4-oxo-3-chinolincarbonsäure werden in einer Schmelze von 20 g 4-Methylpyrazol 6 Stunden auf 130° erhitzt, abgekühlt und mit 100 ml Wasser versetzt. Das ausgefalleneProdukt wird abgesaugt und aus 50 ml Glykolmonomethylether umkristallisiert. Man erhält 2 g (57,7 % der Theorie) 1-Cyclopropyl-6-fluor-1,4-dihydro-7-(3-methyl-1- pyrazolyl)-4-oxo-3-chinolincarbonsäure vom Schmelzpunkt 274-277° (unter Zersetzung).

Beispiel 8

1 g (3,6 mmol) Produkt aus Beispiel J, 0,57 g 3-Oxobutyraldehyd-dimethylacetal und 1 ml konzentrierte Salzsäure werden in 20 ml Ethanol 1 Stunde gekocht. Nach Abkühlung auf Raumtemperatur verdünnt man mit Wasser und isoliert den ausgefallenen Feststoff. Ausbeute: 1,1 g eines Gemisches aus 1-Cyclopropyl-6-fluor-1,4-dihydro-7-(3-methyl-1-pyrazolyl)-4-oxo-3-chinolincarbonsäure und 1-Cyclopropyl-6-fluor-1,4-dihydro-7-(5-methyl-1-pyrazolyl)-4-oxo-3-chinolincarbonsäure vom Schmelzpunkt 221-225° (unter Zersetzung).

Beispie 9

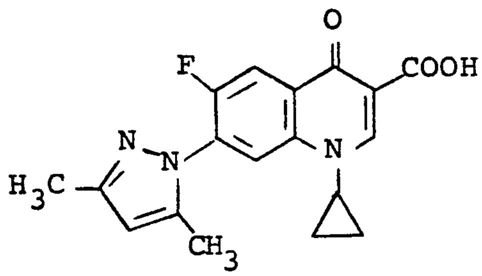

Methode A: Die Herstellung von 1-Cyclopropyl-6-fluor-1,4-dihydro-7-(3,5-dimethyl-1-pyrazolyl)-4-oxo-3-chinolincarbonsäure vom Schmelzpunkt 281-284° (unter Zersetzung) erfolgt analog Beispiel 7 mit 3,5-Dimethylpyrazol.

Methode C: 1 g (3,6 mmol) Produkt aus Beispiel J, 0,43 g Acetylaceton und 1 ml konzentrierte Salzsäure werden in 20 ml Ethanol 1 Stunde gekocht. Nach Abkühlen auf Raumtemperatur wird mit Wasser verdünnt und abgesaugt. Ausbeute: 1,2 g 1-Cyclopropyl-6-fluor-1,4-dihydro-7-(3,5-dimethyl-1-pyrazolyl)-4-oxo-3-chinolincarbonsäure vom Schmelzpunkt 278-280° (unter Zersetzung) (umkristallisiert aus Glykolmonomethylether).

Beispiel 10

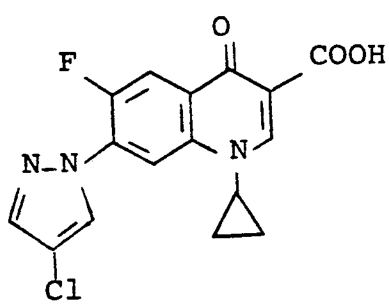

2,65 g (10 mmol) 1-Cyclopropyl-6,7-difluor-1,4-dihydro-4-oxo-3-chinolincarbonsäure werden in 10 ml Dimethylsulfoxid mit 1,03 g 4-Chlorpyrazol und 1,1 g 1,4-Diazabicyclo-[2,2,2]octan (DABCO) versetzt und die Mischung 2 Stunden auf 120° erhitzt. Nach dem Abkühlen wird die Suspension mit 20 ml Wasser verrührt, der Niederschlag abgesaugt und aus Glykolmonomethylether umkristallisiert. Man erhält 2 g (58 % der Theorie) 7-(4-Chlor-1-pyrazolyl)-1-cyclopropyl-6-fluor-1,4-dihydro-4-oxo-3-chinolincarbonsäure vom Schmelzpunkt 295-296° (unter Zersetzung).

Beispiel 11

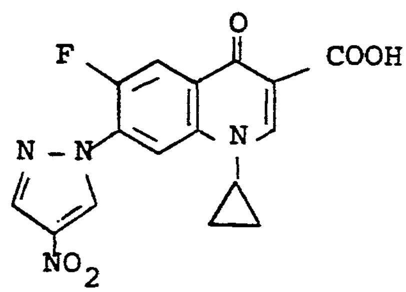

Analog Beispiel 10 setzt man 4-Nitropyrazol während 4 Stunden bei 120° um und erhält 1-Cyclopropyl-6-fluor-1,4- dihydro-7-(4-nitro-1-pyrazolyl)-4-oxo-3-chinolin-carbonsäure vom Schmelzpunkt 328° (unter Zersetzung).

Beispiel 12

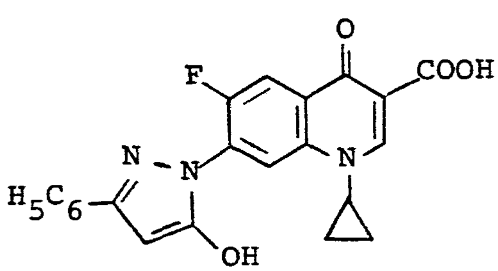

3 g (10,8 mmol) Produkt aus Beispiel J und 2,5 g Benzoylessigsäureethylester werden in 40 ml Eisessig 8 Stunden gekocht. Nach Abkühlen wird mit Wasser verdünnt und der Feststoff isoliert. Ausbeute: 3,1 g (71 % der Theorie) 1-Cyclopropyl-6-fluor-1,4-dihydro-7-(5-hydroxy-3-phenyl-1-pyrazolyl)-4-oxo-3-chinolincarbonsäure vom Schmelzpunkt 301-303° (unter Zersetzung).

Beispiel 13

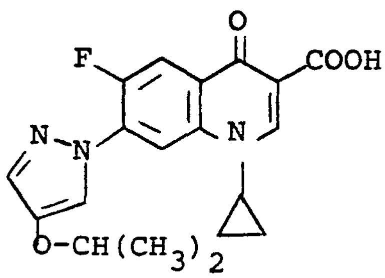

3 g (10,8 mmol) Produkt aus Beispiel J, 2,1 g 3-Dimethylamino-2-isopropoxy-acrolein und 1 ml konzentrierte Salzsäure werden in 20 ml Ethanol 30 Minuten gekocht. Nach Abkühlen wird mit Wasser verdünnt und abgesaugt. Ausbeute: 3,7 g (92 % der Theorie) 1-Cyclopropyl-6-fluor-1,4-dihydro-7-(4-isopropoxy-1-pyrazolyl)-4-oxo-3-chinolincarbonsäure vom Schmelzpunkt 219-221° (unter Zersetzung).

Beispiel 14

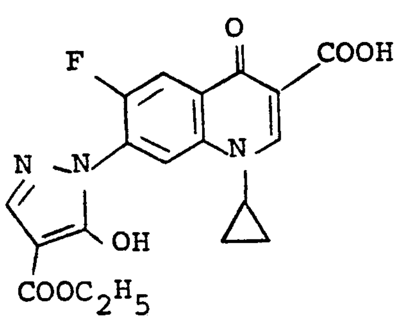

3 g (10,8 mmol) Produkt aus Beispiel J und 10 ml Ethoxymethylenmalonsäurediethylester werden 1 Stunde auf 80°C erhitzt. Nach Abkühlen verdünnt man mit Ethanol und isoliert den ausgefallenen Feststoff. Ausbeute: 4,2 g (97 % der Theorie) 1-Cyclopropyl-7-(4-ethoxycarbonyl-5-hydroxy-1-pyrazolyl)- 6-fluor-1,4-dihydro-4-oxo-3-chinolincarbonsäure vom Schmelzpunkt 218-219° (unter Zersetzung).

Beispiel 15

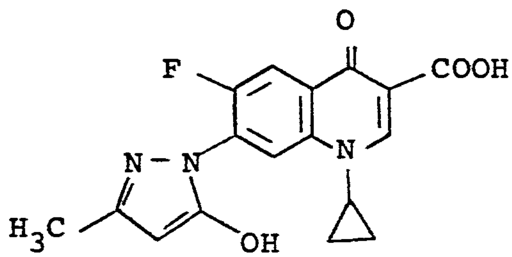

3 g (10,8 mmol) Produkt aus Beispiel J und 1,7 g Acetessigsäureethylester werden in 40 ml Ethanol 8 Stunden erhitzt. Danach wird im Vakuum eingeengt. Der Rückstand wird mit 0,6 g NaOH in 30 ml Ethanol-Wasser-Gemisch (1:1) 2 Stunden gekocht. Nach Abkühlung wird mit verdünnter Salzsäure angesäuert und mit Methylenchlorid extrahiert. Nach Trocknen über $Na_2SO_4$ wird die organische Phase eingedampft. Der Rückstand wird mit Acetonitril verrührt und der entstehende Feststoff isoliert. Ausbeute: 1,5 g (40 % der Theorie) 1-Cyclopropyl- 6-fluor-1,4-dihydro-7-(5-hydroxy-3-methyl-1-pyrazolyl)- 4-oxo-3-chinolincarbonsäure vom Schmelzpunkt 230-232° (unter Zersetzung).

**Beispiel 16**

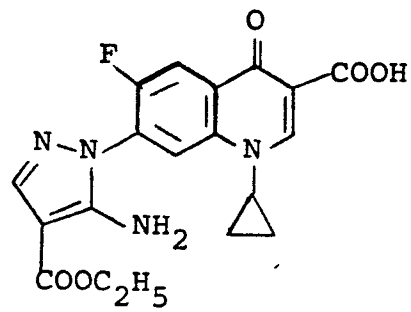

1,5 g (5,4 mmol) Produkt aus Beispiel J und 1 g Ethoxymethylencyanessigsäureethylester werden in 30 ml Ethanol 4 Stunden gekocht. Nach Abkühlung wird der Feststoff abgesaugt, getrocknet und in 15 ml konzentrierter Schwefelsäure gelöst. Man rührt eine Stunde bei Raumtemperatur und gießt dann auf Eis. Der ausgefallene Feststoff wird isoliert und getrocknet. Ausbeute: 1,2 g (56 % der Theorie) 7-(5-Amino-4-ethoxycarbonyl-1-pyrazolyl)-1-cyclopropyl-6-fluor-1,4-dihydro-4-oxo-3-chinolincarbonsäure vom Schmelzpunkt 290-292° (unter Zersetzung).

**Beispiel 17**

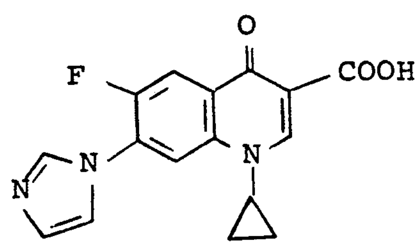

2,65 g (10 mmol) 1-Cyclopropyl-6,7-difluor-1,4-dihydro-4-oxo-3-chinolincarbonsäure werden in einer Schmelze von 20 g Imidazol 2 Stunden auf 130° erhitzt und überschüssiges Imidazol anschließend im Hochvakuum abdestilliert. Der Rückstand wird mit 50 ml Wasser versetzt, mit konzentrierter Salzsäure auf pH 6-7 eingestellt, der ausgefallene Niederschlag abgesaugt und aus Glykolmonomethylether umkristallisiert. Man erhält 1,5 g (48 % der Theorie) 1-Cyclopropyl-6-fluor-1,4-dihydro-7-(1-imidazolyl)-4-oxo-3-chinolincarbonsäure vom Schmelzpunkt 288-291° (unter Zersetzung).

Beispiel 18

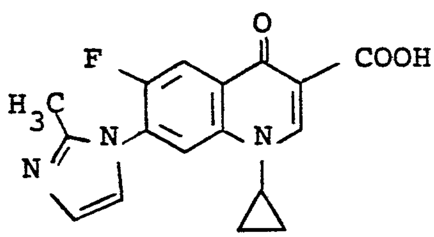

Man verfährt analog Beispiel 7 mit 2-Methylimidazol als Ausgangsprodukt und stellt das Reaktionsgemisch bei der Aufarbeitung mit Salzsäure auf pH 6, wobei 1-Cyclopropyl-6-fluor-1,4-dihydro-7-(2-methyl-1-imidazolyl)-4-oxo-3-chinolincarbonsäure vom Schmelzpunkt 294-297° (unter Zersetzung) (umkristallisiert aus Glykolmonomethylether) erhalten werden.

Beispiel 19

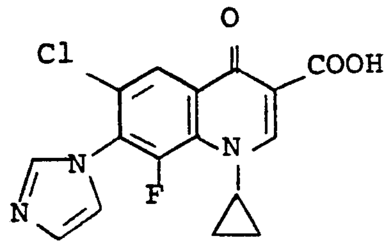

Analog Beispiel 17 wird 6-Chlor-1-cyclopropyl-7,8-difluor-1,4-dihydro-4-oxo-3-chinolincarbonsäure mit Imidazol umgesetzt und 6-Chlor-1-cyclopropyl-8-fluor-1,4-dihydro-7-(1-imidazolyl)-4-oxo-3-chinolincarbonsäure vom Schmelzpunkt 212-215° (unter Zersetzung) erhalten.

Beispiel 20

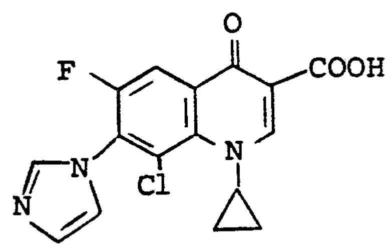

Analog Beispiel 17 wird 8-Chlor-1-cyclopropyl-6,7-difluor-1,4-dihydro-4-oxo-3-chinolincarbonsäure mit Imidazol umgesetzt und 8-Chlor-1-cyclopropyl-6-fluor-1,4-dihydro-7-(1-imidazolyl)-4-oxo-3-chinolincarbonsäure vom Schmelzpunkt 258-260° (unter Zersetzung) isoliert.

Beispiel 21

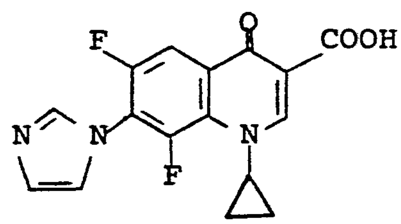

2,72 g(9,6 mmol) 1-Cyclopropyl-6,7,8-trifluor-1,4-dihydro-4-oxo-3-chinolincarbonsäure werden in 16 ml Dimethylformamid mit 2 g Imidazol 75 Minuten auf 100° erhitzt. Man läßt über Nacht stehen, saugt den ausgefallenen Niederschlag ab, wäscht mit Ethanol und kristallisiert aus Dimethylformamid um. Man erhält 1,5 g (47 % der Theorie) 1-Cyclopropyl-6,8-difluor-1,4-dihydro-7-(1-imidazolyl)-4-oxo-3-chinolincarbonsäure vom Schmelzpunkt 253-254° (unter Zersetzung).

Beispiel 22

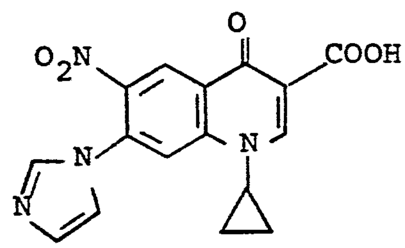

Analog Beispiel 17 setzt man 7-Chlor-1-cyclopropyl-1,4-dihydro-6-nitro-4-oxo-3-chinolincarbonsäure mit Imidazol um und erhält 1-Cyclopropyl-1,4-dihydro-7-(1-imidazolyl)-6-nitro-4-oxo-3-chinolincarbonsäure vom Schmelzpunkt 266-267° (unter Zersetzung).

**Beispiel 23**

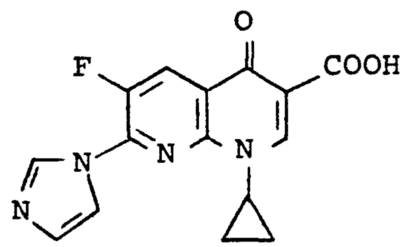

Analog Beispiel 17 wird 7-Chlor-1-cyclopropyl-6-fluor-1,4-dihydro-4-oxo-1,8-naphthyridin-3-carbonsäure mit Imidazol umgesetzt und 1-Cyclopropyl-6-fluor-1,4-dihydro-7-(1-imidazolyl)-4-oxo-1,8-naphthyridin-3-carbonsäure erhalten.

**Beispiel 24**

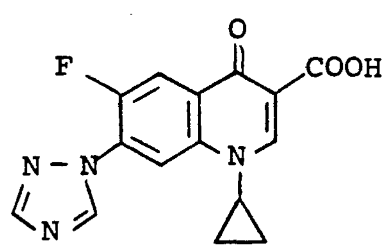

Analog Beispiel 7 wird die Umsetzung in einer Schmelze von 1,2,4-Triazol während 2 Stunden bei 130° durchgeführt und nach der Aufarbeitung 1-Cyclopropyl-6-fluor-1,4-dihydro-4-oxo-7-(1,2,4-triazol-1-yl)-3-chinolincarbonsäure vom Schmelzpunkt 307-311° (unter Zersetzung) isoliert.

Beispiel 25

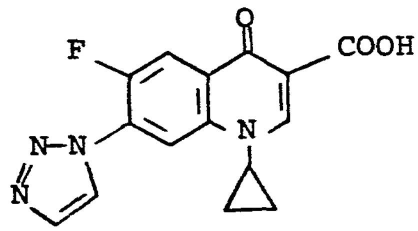

Analog Beispiel 7 wird die Umsetzung mit 1,2,3-Triazol während 4 Stunden bei 130°C durchgeführt und nach der Aufarbeitung 1-Cyclopropyl-6-fluor-1,4-dihydro-4-oxo-7-(1,2,3-triazol-1-yl)-3-chinolincarbonsäure vom Schmelzpunkt 235-241° (unter Zersetzung) isoliert.

Beispiel 26

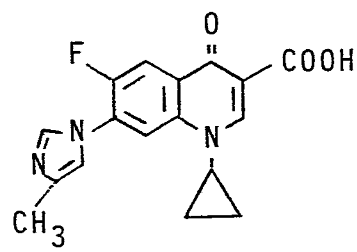

2,65 g (10 mmol) 1-Cyclopropyl-6,7-difluor-1,4-dihydro-4-oxo-3-chinolincarbonsäure werden in 10 ml Dimethylsulfoxid mit 0,9 g (11 mmol) 4-Methylimidazol und 1,1 g 1,4-Diazabicyclo/2,2,2/octan 3 Stunden auf 120° erhitzt. Die Suspension wird mit 50 ml Wasser verrührt, der Niederschlag abgesaugt, mit Wasser gewaschen, getrocknet und aus Glykolmonomethylether umkristallisiert.

Ausbeute: 1,4 g vom Schmelzpunkt 274° (unter Zersetzung).

## Patentansprüche

1. Verbindungen der Formel (I),

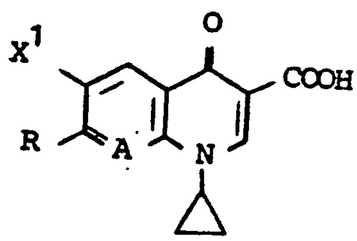

(I)

in welcher

$X^1$ für Halogen oder Nitro steht,

A für Stickstoff oder $C-X^2$ steht, worin

$X^2$ Wasserstoff oder Halogen bedeutet, und

R für einen über Stickstoff gebundenen, gegebenenfalls durch Alkyl mit 1 bis 4 Kohlenstoffatomen, Alkoxy mit 1 bis 4 Kohlenstoffatomen, Phenyl, Halogen, wie Fluor, Chlor oder Brom, Nitro, Amino, Hydroxy, Cyano, Ethoxycarbonyl oder Carboxy ein- bis dreifach substituierten Azolyl-Rest, wie für einen Pyrrolyl-, Pyrazolyl-, Imidazolyl-, 1,2,3-Triazolyl- oder 1,2,4-Triazolyl-Rest steht,

und deren pharmazeutisch verwendbaren Hydrate, Säureadditionssalze, Alkali- und Erdalkalisalze, sowie deren Prodrug-Formen.

2. Verbindungen der Formel (I) nach Anspruch 1

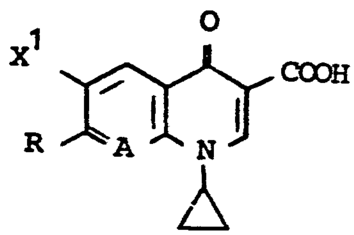

(I)

in welcher

$X^1$ für Fluor, Chlor, Brom oder Nitro steht,

A für Stickstoff oder C-$X^2$ steht, worin

$X^2$ Wasserstoff, Fluor, Chlor oder Brom bedeutet, und

R für einen über Stickstoff gebundenen, gegebenenfalls durch Alkyl mit 1 bis 4 Kohlenstoffatomen, Alkoxy mit 1 bis 4 Kohlenstoffatomen, Phenyl, Halogen, wie Fluor, Chlor oder Brom, Nitro, Amino, Hydroxy, Cyano, Ethoxycarbonyl oder Carboxy ein- bis dreifach substituierten Azolyl-Rest steht,

und deren pharmazeutisch verwendbaren Hydrate, Säureadditionssalze, Alkali- und Erdalkalisalze, sowie deren Prodrug-Formen.

3. Verbindungen der Formel (I) nach Anspruch 1, in denen

$X^1$ für Fluor, Chlor oder Nitro steht,

A für Stickstoff oder C-$X^2$ steht, worin

$X^2$ Wasserstoff, Fluor oder Chlor bedeutet, und

R für einen über Stickstoff gebundenen, gegebenenfalls durch Methyl, Ethyl, Alkoxy mit 1 bis 3 Kohlenstoffatomen, Phenyl, Chlor, Nitro, Amino, Hydroxy oder Ethoxycarbonyl ein- oder zweifach substituierten Azolyl-Rest, wie für einen Pyrrolyl-, Pyrazolyl-, Imidazolyl-, 1,2,3-Triazolyl- oder 1,2,4-Triazolyl-Rest, steht,

und deren pharmazeutisch verwendbaren Hydrate, Säureadditionssalze, Alkali- und Erdalkalisalze, sowie deren Prodrug-Formen, wie Methyl-, Ethyl- oder (2-Methoxyethyl)-ester.

4. Verbindungen der Formel (I) gemäß Anspruch 1, in denen

$X^1$ für Fluor, Chlor oder Nitro steht,

A für C-$X^2$ steht, worin

$X^2$ Wasserstoff, Fluor oder Chlor bedeutet, und

R für einen über Stickstoff gebundenen, gegebenenfalls durch Methyl, Alkoxy mit 1 bis 3 Kohlenstoffatomen, Phenyl, Chlor, Nitro, Amino, Hydroxy oder Ethoxycarbonyl ein- oder zweifach substituierten Azolyl-Rest, wie für einen Pyrrolyl-, Pyrazolyl-, Imidazolyl-, 1,2,3-Triazolyl- oder 1,2,4-Triazolyl-Rest, steht,

und deren pharmazeutisch verwendbaren Hydrate, Säureadditionssalze, Alkali- und Erdalkalisalze, sowie deren Prodrug-Formen, wie Methyl-, Ethyl- oder (2-Methoxyethyl)-ester.

5. Verbindungen aus der Gruppe bestehend aus
6-Chlor-1-cyclopropyl-8-fluor-1,4-dihydro-4-oxo-7-(1-pyrrolyl)-3-chinolincarbonsäure,
8-Chlor-1-cyclopropyl-6-fluor-1,4-dihydro-4-oxo-7-(1-pyrrolyl)-3-chinolincarbonsäure,
6,8-Dichlor-1-cyclopropyl-1,4-dihydro-4-oxo-7-(1-pyrrolyl)-3-chinolincarbonsäure,
1-Cyclopropyl-6-fluor-1,4-dihydro-4-oxo-7-(1-pyrrolyl)-1,8-naphthyridin-3-carbonsäure,
6-Chlor-1-cyclopropyl-1,4-dihydro-4-oxo-7-(1-pyrrolyl)-1,8-naphthyridin-3-carbonsäure und
6-Chlor-1-cyclopropyl-1,4-dihydro-7-(1-imidazolyl)-4-oxo-1,8-naphthyridin-3-carbonsäure.

6. Verbindungen aus der Gruppe bestehend aus

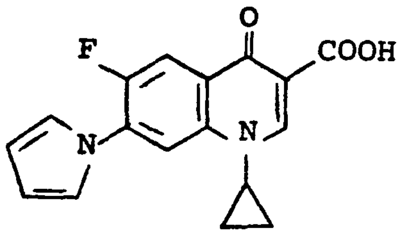

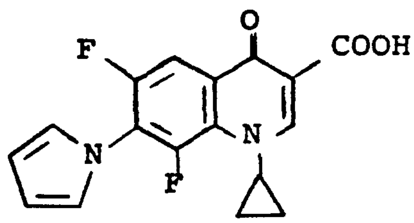

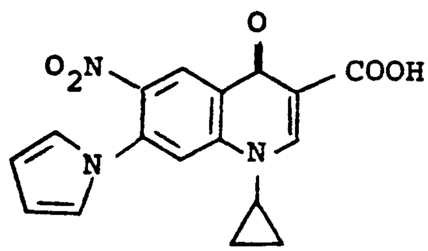

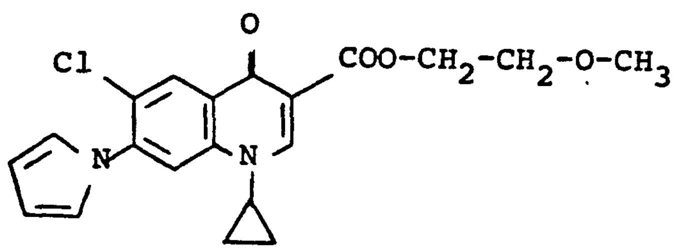

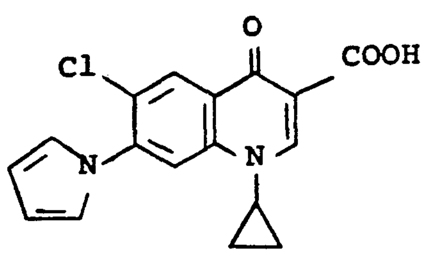

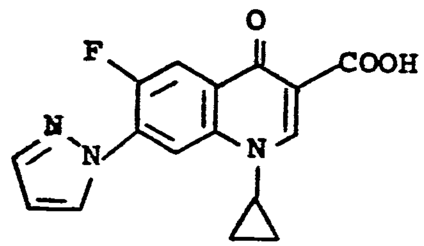

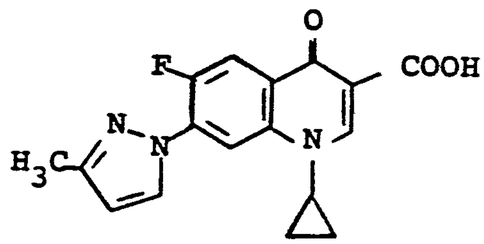

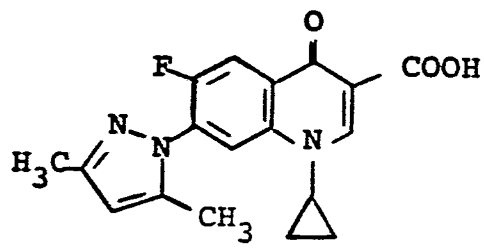

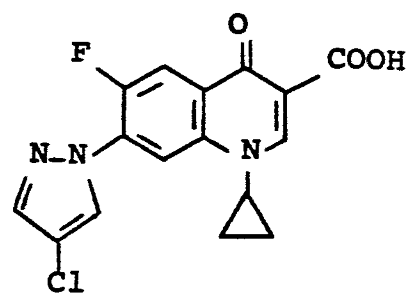

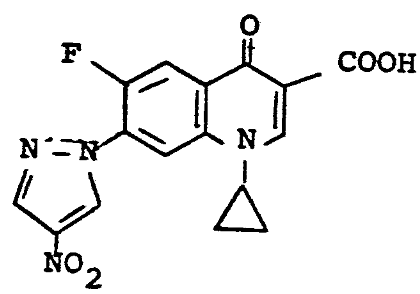

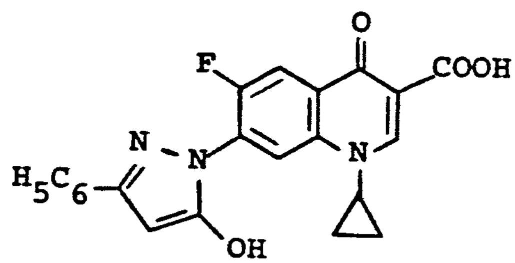

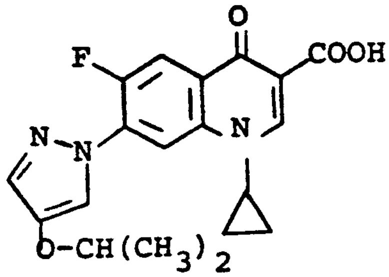

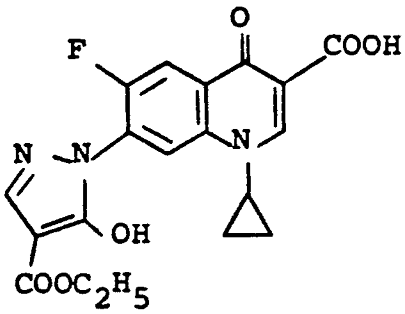

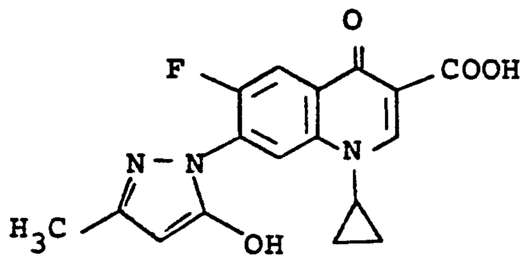

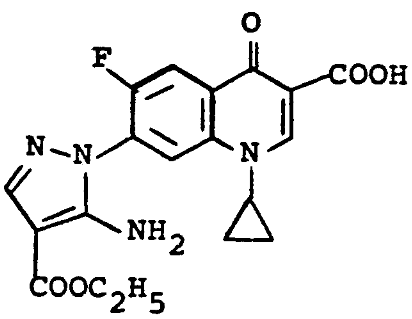

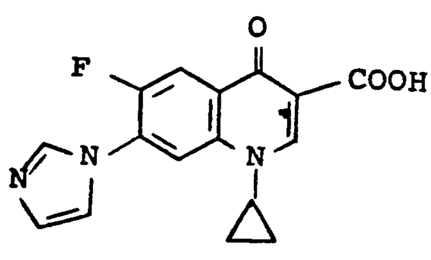

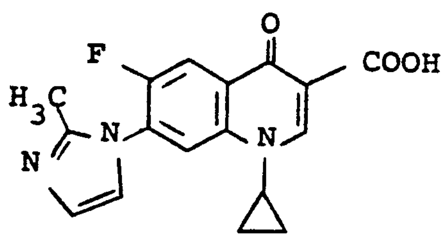

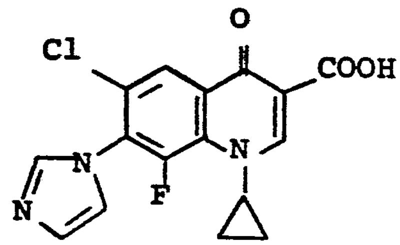

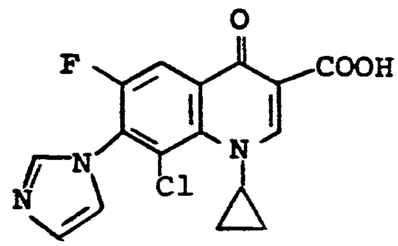

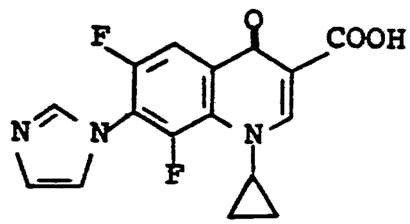

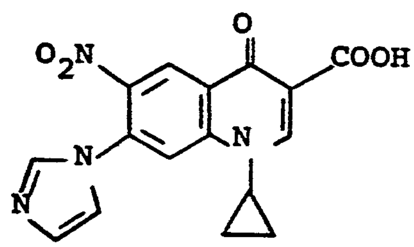

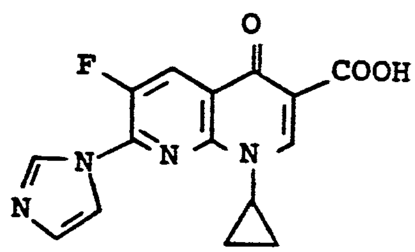

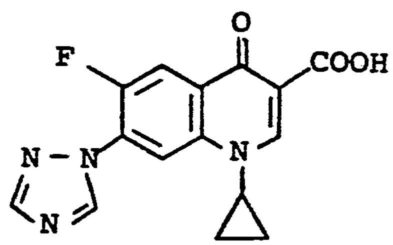

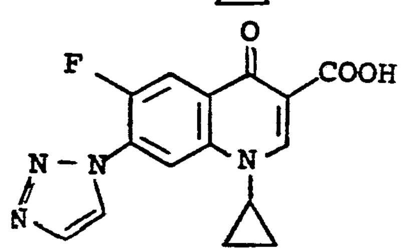

7. Verbindungen aus der Gruppe bestehend aus

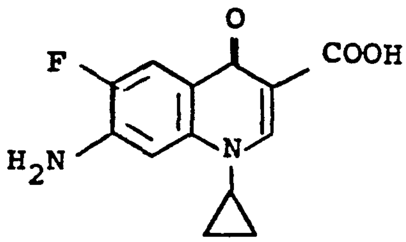

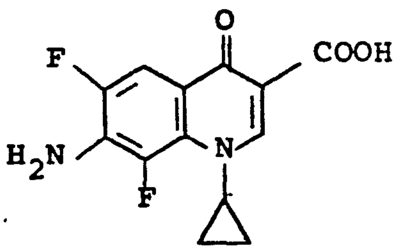

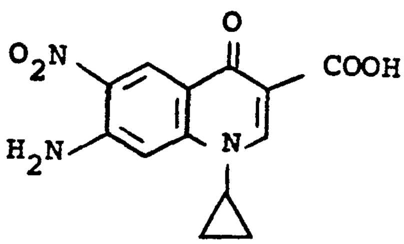

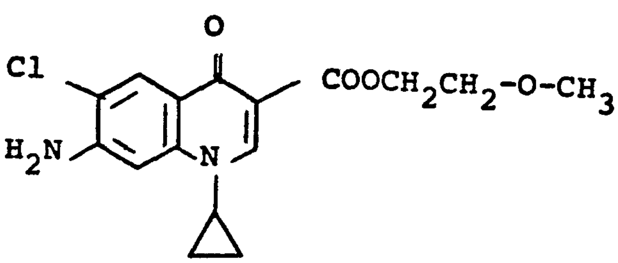

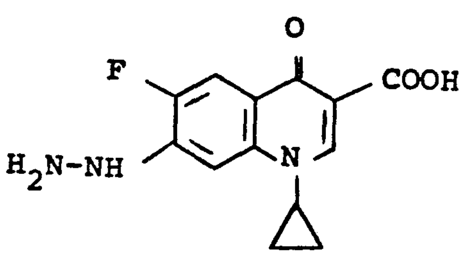

8. Verfahren zur Herstellung von Verbindungen der Formel (I),

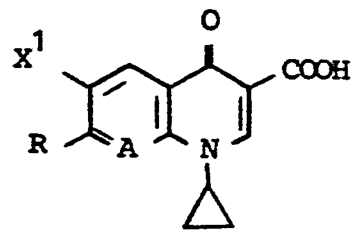

(I)

in welcher

$X^1$ für Halogen, wie Fluor, Chlor oder Brom, oder Nitro steht,

A für Stickstoff oder $C-X^2$ steht, worin

$X^2$ Wasserstoff oder Halogen, wie Fluor, Chlor oder Brom bedeutet, und

R für einen über Stickstoff gebundenen, gegebenenfalls durch Alkyl mit 1 bis 4 Kohlenstoffatomen, Alkoxy mit 1 bis 4 Kohlenstoffatomen, Phenyl, Halogen, wie Fluor, Chlor oder Brom, Nitro, Amino, Hydroxy, Cyano, Ethoxycarbonyl oder Carboxy ein- bis dreifach substituierten Azolyl-Rest, wie für einen Pyrrolyl-, Pyrazolyl-, Imidazolyl-, 1,2,3-Triazolyl- oder 1,2,4-Triazolyl-Rest steht,

und deren pharmazeutisch verwendbaren Hydrate, Säureadditionssalze, Alkali- und Erdalkalisalze, sowie deren Prodrug-Formen, wie Methyl-, Ethyl- oder (2-Methoxyethyl)-ester, dadurch gekennzeichnet, daß

Verbindungen der Formel (II),

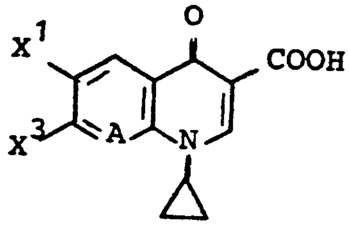

(II)

in welcher

A und $X^1$ die oben angegebene Bedeutung haben und

$X^3$ für Fluor oder Chlor steht,

mit Azolen der Formel (III),

R - H (III)

in welcher

R die oben angegebene Bedeutung hat,

gegebenenfalls in Gegenwart von Säurebindern umgesetzt werden und die erhaltenen Verbindungen gegebenenfalls in Hydrate, Säureadditionssalze, Alkali- und Erdalkalisalze oder Prodrug-Formen überführt werden.

9. Verfahren zur Herstellung von Verbindungen der Formel (I)

0203488

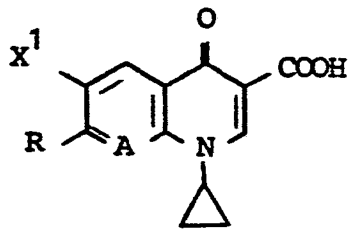

(I)

in welcher

$X^1$ für Halogen, wie Fluor, Chlor oder Brom, oder Nitro steht,

A für Stickstoff oder C-$X^2$ steht, worin

$X^2$ Wasserstoff oder Halogen, wie Fluor, Chlor oder Brom bedeutet, und

R für einen über Stickstoff gebundenen, gegebenenfalls durch Methyl oder Ethyl ein- oder zweifach substituierten Pyrrolylrest steht,

und deren pharmazeutisch verwendbaren Hydrate, Säureadditionssalze, Alkali- und Erdalkalisalze, sowie deren Prodrug-Formen, dadurch gekennzeichnet, daß man Verbindungen der Formel (IV)

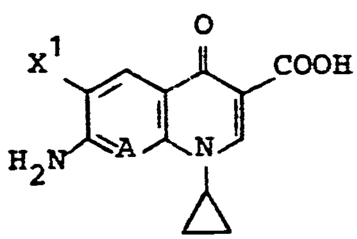

(IV)

in welcher

A und $X^1$ die oben angegebene Bedeutung haben,

mit 1,4-Dioxoverbindungen der Formel (V)

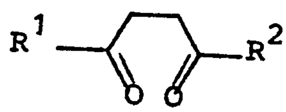

(V)

in welcher

$R^1$ und $R^2$ gleich oder verschieden sind und für Wasserstoff, Methyl oder Ethyl stehen,

bzw. mit verkappten 1,4-Dioxoverbindungen der Formel (VI)

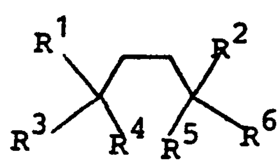

(VI)

in welcher

$R^1$ und $R^2$ die oben angegebene Bedeutung haben und

$R^3$,$R^4$,$R^5$ und $R^6$ gleich oder verschieden sind und für Methoxy, Ethoxy oder Chlor stehen oder $R^4$ und $R^5$ gemeinsam eine Sauerstoffbrücke bilden können,

umsetzt und die erhaltenen Verbindungen gegebenenfalls in Hydrate, Säureadditionssalze, Alkali- und Erdalkalisalze oder Prodrug-Formen überführt.

10. Verfahren zur Herstellung von Verbindungen der Formel (I) nach Anspruch 8, in welcher R für einen über Stickstoff verknüpften Pyrazolrest steht, dadurch gekennzeichnet, daß man eine Hydrazinocarbonsäure der Formel (VII)

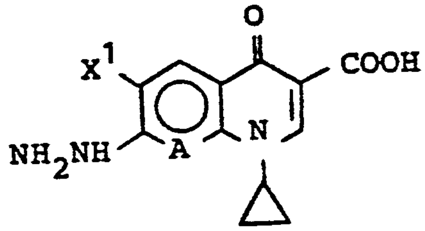

(VII)

in welcher

A und $X^1$ die in Anspruch 8 angegebene Bedeutung haben,

mit 1,3-Dicarbonylverbindungen der Formel (VIII)

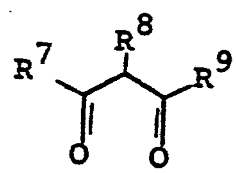

(VIII)

in welcher

$R^7$,$R^8$ und $R^9$ gleich oder verschieden sind und für Wasserstoff, Methyl, Ethyl, Methoxy oder Ethoxy stehen,

bzw. mit verkappten 1,3-Dicarbonylverbindungen der Formel (IX),

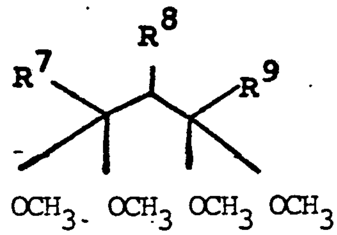

(IX)

in welcher

$R^7$,$R^8$ und $R^9$ die oben angegebene Bedeutung haben,

oder mit verkappten 1,3-Dicarbonylverbindungen der Formel (X),

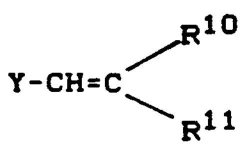

(X)

in welcher

Y für Dimethylamino, Methoxy oder Ethoxy,

$R^{10}$ für Formyl, Methoxycarbonyl, Ethoxycarbonyl oder Cyano und

$R^{11}$ für Wasserstoff, Alkyl mit 1 bis 4 Kohlenstoffatomen, Alkoxy mit 1 bis 4 Kohlenstoffatomen, Methoxycarbonyl, Ethoxycarbonyl oder Cyano steht, umsetzt und die erhaltenen Verbindungen gegebenenfalls in Hydrate, Säureadditionssalze, Alkali- und Erdalkalisalze oder Prodrug-Formen überführt.

11. Arzneimittel enthaltend Verbindungen der Formel (I)

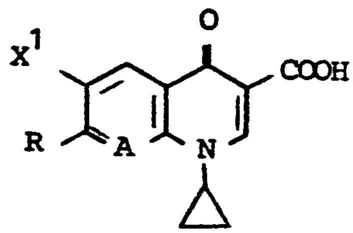

(I)

in welcher

$X^1$ für Halogen, wie Fluor, Chlor oder Brom, oder Nitro steht,

A für Stickstoff oder $C-X^2$ steht, worin

$X^2$ Wasserstoff oder Halogen, wie Fluor, Chlor oder Brom bedeutet, und

R für einen über Stickstoff gebundenen, gegebenenfalls durch Alkyl mit 1 bis 4 Kohlenstoffatomen, Alkoxy mit 1 bis 4 Kohlenstoffatomen, Phenyl, Halogen, wie Fluor, Chlor oder Brom, Nitro, Amino, Hydroxy, Cyano, Ethoxycarbonyl oder Carboxy ein- bis dreifach substituierten Azolyl-Rest, wie für einen Pyrrolyl-, Pyrazolyl-, Imidazolyl-, 1,2,3-Triazolyl- oder 1,2,4-Triazolyl-Rest steht,

und deren pharmazeutisch verwendbare Hydrate, Säureadditionssalze, Alkali- und Erdalkalisalze, sowie deren Prodrug-Formen, wie Methyl-, Ethyl- oder (2-Methoxyethyl)-ester.

 

12. Verwendung von Verbindungen der Formel (I)

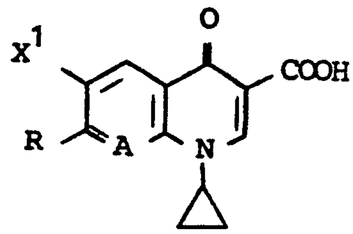

(I)

in welcher

$X^1$ für Halogen, wie Fluor, Chlor oder Brom, oder Nitro steht,

A für Stickstoff oder C-$X^2$ steht, worin

$X^2$ Wasserstoff oder Halogen, wie Fluor, Chlor oder Brom bedeutet, und

R für einen über Stickstoff gebundenen, gegebenenfalls durch Alkyl mit 1 bis 4 Kohlenstoffatomen, Alkoxy mit 1 bis 4 Kohlenstoffatomen, Phenyl, Halogen, wie Fluor, Chlor oder Brom, Nitro, Amino, Hydroxy, Cyano, Ethoxycarbonyl oder Carboxy ein- bis dreifach substituierten Azolyl-Rest, wie für einen Pyrrolyl-, Pyrazolyl-, Imidazolyl-, 1,2,3-Triazolyl- oder 1,2,4-Triazolyl-Rest steht,

und deren pharmazeutisch verwendbare Hydrate, Säureadditionssalze, Alkali- und Erdalkalisalze, sowie deren Prodrug-Formen, wie Methyl-, Ethyl- oder (2-Methoxyethyl)-ester zur Herstellung von Arzneimitteln.

**13.** Verbindungen der Formel (I)

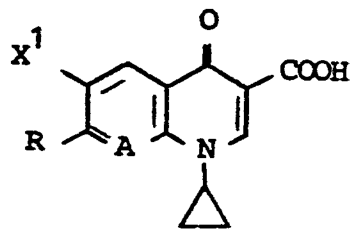

(I)

in welcher

$X^1$ für Halogen, wie Fluor, Chlor oder Brom, oder Nitro steht,

A für Stickstoff oder $C-X^2$ steht, worin

$X^2$ Wasserstoff oder Halogen, wie Fluor, Chlor oder Brom bedeutet, und

R für einen über Stickstoff gebundenen, gegebenenfalls durch Alkyl mit 1 bis 4 Kohlenstoffatomen, Alkoxy mit 1 bis 4 Kohlenstoffatomen, Phenyl, Halogen, wie Fluor, Chlor oder Brom, Nitro, Amino, Hydroxy, Cyano, Ethoxycarbonyl oder Carboxy ein- bis dreifach substituierten Azolyl-Rest, wie für einen Pyrrolyl-, Pyrazolyl-, Imidazolyl-, 1,2,3-Triazolyl- oder 1,2,4-Triazolyl-Rest steht,

und deren pharmazeutisch verwendbare Hydrate, Säureadditionssalze, Alkali- und Erdalkalisalze, sowie deren Prodrug-Formen, wie Methyl-, Ethyl- oder (2-Methoxyethyl)-ester zur Anwendung in einem Verfahren zur therapeutischen Behandlung des menschlichen oder tierischen Körpers.

14. Verwendung von Verbindungen

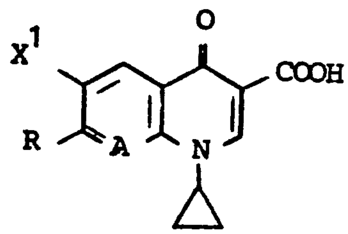

(I)

in welcher

$X^1$ für Halogen, wie Fluor, Chlor oder Brom, oder Nitro steht,

A für Stickstoff oder C-$X^2$ steht, worin

$X^2$ Wasserstoff oder Halogen, wie Fluor, Chlor oder Brom bedeutet, und

R für einen über Stickstoff gebundenen, gegebenenfalls durch Alkyl mit 1 bis 4 Kohlenstoffatomen, Alkoxy mit 1 bis 4 Kohlenstoffatomen, Phenyl, Halogen, wie Fluor, Chlor oder Brom, Nitro, Amino, Hydroxy, Cyano, Ethoxycarbonyl oder Carboxy ein- bis dreifach substituierten Azolyl-Rest, wie für einen Pyrrolyl-, Pyrazolyl-, Imidazolyl-, 1,2,3-Triazolyl- oder 1,2,4-Triazolyl-Rest steht,

und deren pharmazeutisch verwendbare Hydrate, Säureadditionssalze, Alkali- und Erdalkalisalze, sowie deren Prodrug-Formen, wie Methyl-, Ethyl- oder (2-Methoxyethyl)-ester zur qualitativen und quantitativen Verbesserung tierischer Erzeugnisse.

15. Futtermittel, Futterzusatzmittel und Prämixe zur Verwendung in der Tierernährung, enthaltend Verbindungen der Formel (I) gemäß Anspruch 1.